(19) Europäisches Patentamt
European Patent Office
Office européen des brevets

(11) Publication number: **0 630 897 A2**

(12) # EUROPEAN PATENT APPLICATION

(21) Application number: **94109034.2**

(22) Date of filing: **13.06.94**

(51) Int. Cl.5: **C07D 473/04**, C07D 239/54, C07D 487/04, //(C07D487/04, 249:00,239:00)

(30) Priority: **25.06.93 US 81718**

(43) Date of publication of application: **28.12.94 Bulletin 94/52**

(84) Designated Contracting States: **AT BE CH DE DK ES FR GB GR IE IT LI LU MC NL PT SE**

(71) Applicant: **BRISTOL-MYERS SQUIBB COMPANY**
**P.O. Box 4000**
**Princeton, NJ 08543-4000 (US)**

(72) Inventor: **Tino, Joseph A.**
**11 Chopin Lane**
**Lawrenceville, NJ (US)**
Inventor: **Zahler, Robert**
**5 East Welling Avenue**
**Pennington, NJ (US)**

(74) Representative: **Josif, Albert, Dr.-Ing. et al**
**Baaderstrasse 3**
**D-80469 München (DE)**

(54) **3-Hydroxy-4-hydroxymethyl-2-methylene-cyclopentyl purines and pyrimidines.**

(57) Antiviral activity is exhibited by compounds having the formula

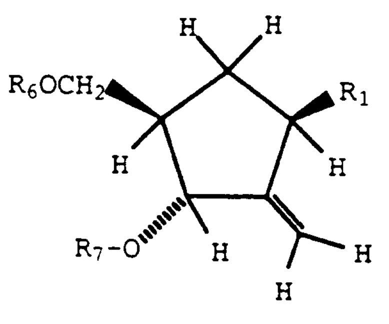

or a pharmaceutically acceptable salt thereof.

EP 0 630 897 A2

Zahler et al. in European Patent Application 481,754 disclose 4-hydroxy-3-hydroxymethyl-2-methylenecyclopentyl purines and pyrimidines of the formula

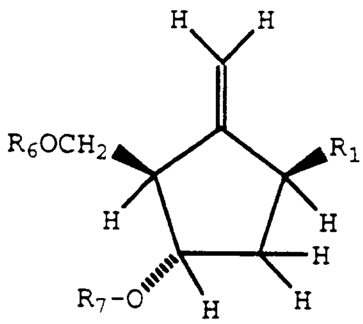

wherein $R_1$ is a substituted purinyl or pyrimidinyl moiety.

Antiviral activity is exhibited by compounds having the formula

1

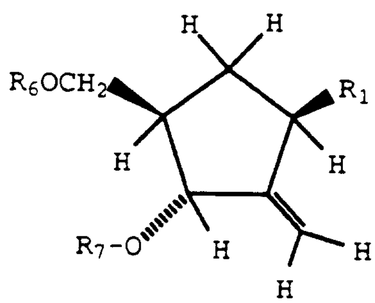

and its pharmaceutically acceptable salts. In formula 1, and throughout the specification, the symbols are as defined below.

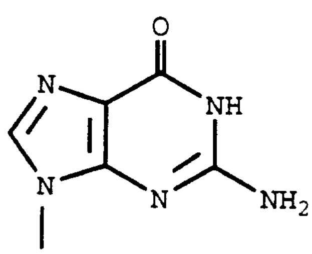

,

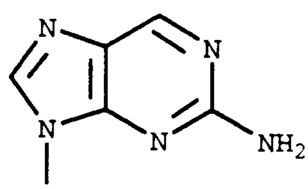

,

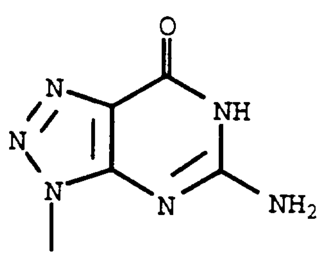

,

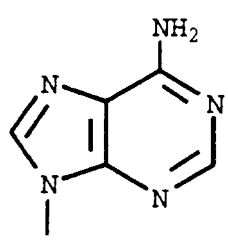

,

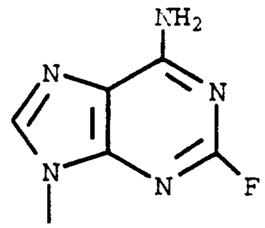 , 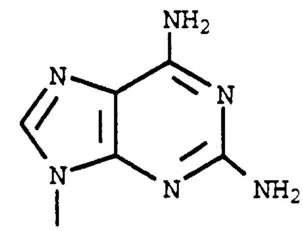 ,

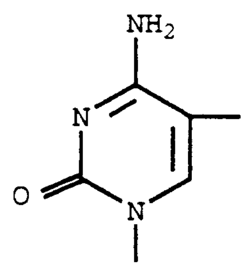 , 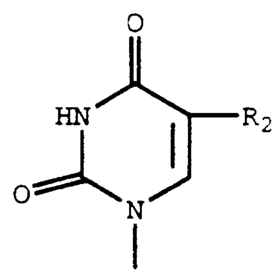 ,

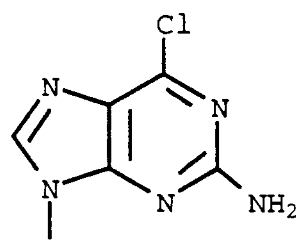 , 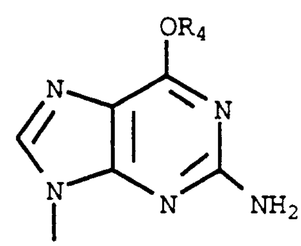 ,

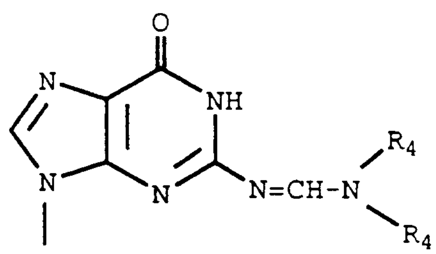 , 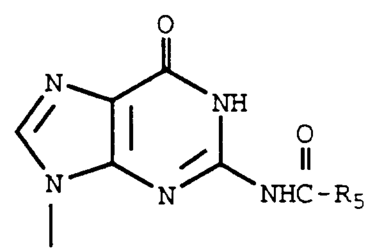 ,

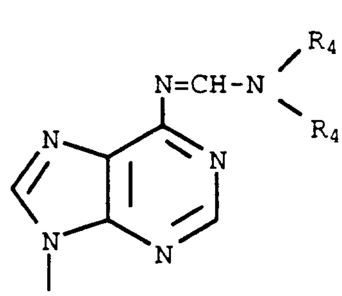 , 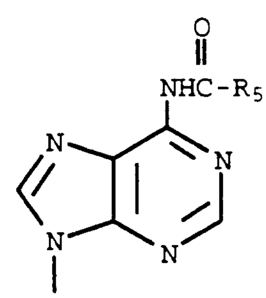 ,

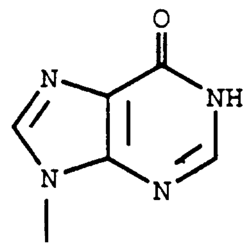 , or 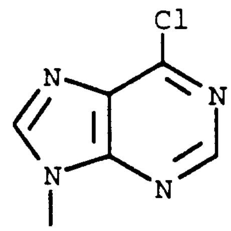 .

$R_2$ is fluoro, chloro, bromo, iodo, hydrogen, methyl, trifluoromethyl, ethyl, n-propyl, 2-fluoroethyl, 2-chloroethyl, ethynyl, or

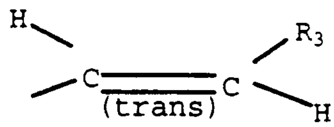 .

$R_3$ is chloro, bromo, iodo, hydrogen, methyl or trifluoromethyl.
$R_4$ is alkyl.
$R_5$ is hydrogen, alkyl, substituted alkyl or aryl.
$R_6$ and $R_7$ are independently selected from hydrogen, $-PO_3H_2$, and

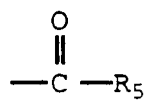 .

Preferred compounds of formula 1 are those wherein
$R_1$ is

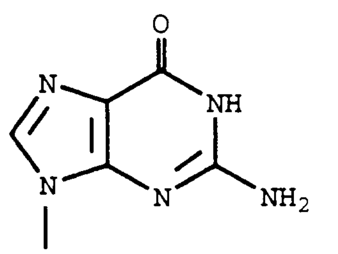 , 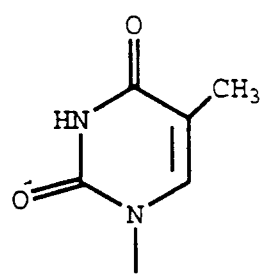 , or

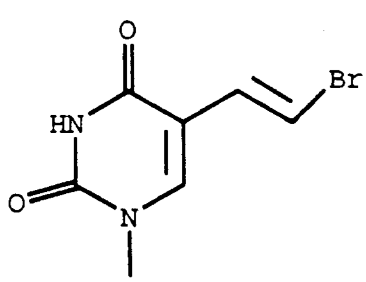 .

The term "alkyl" refers to both straight and branched chain groups. Those groups having 1 to 10 carbons are preferred. The term "substituted alkyl" refers to alkyl groups having one or more, preferably one, substituents. Preferred substituents are halogen, amino, azido, hydroxy, cyano, trialkylammonium

(wherein each alkyl group has 1 to 6 carbons), alkoxy of 1 to 6 carbons, aryl and carboxy. The term "aryl" refers to phenyl and phenyl substituted with one, two or three substituents, preferably one. Preferred substituents are alkyl of 1 to 6 carbons, alkoxy of 1 to 6 carbons, halogen, trifluoromethyl, amino, alkylamino of 1 to 6 carbons, dialkylamino wherein each alkyl is of 1 to 6 carbons, nitro, cyano, alkanoyloxy of 2 to 11 carbons, carboxy, carbamoyl and hydroxy.

The compounds of formula 1, and the pharmaceutically acceptable salts thereof, are antiviral agents that can be used to treat viral infections in mammalian species such as domesticated animals (e.g., dogs, cats, horses and the like) and humans, and avian species (e.g., chickens and turkeys). The compounds of formula 1 wherein $R_1$ is

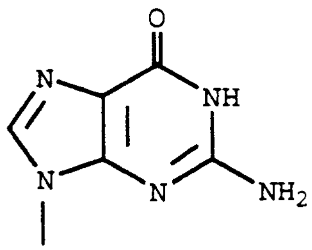
,
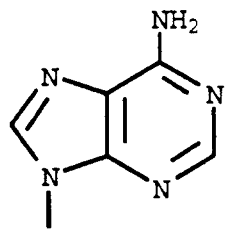
,
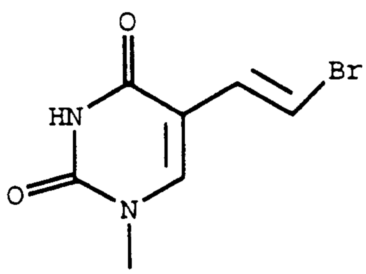
,
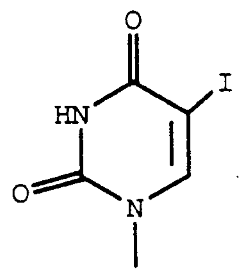
, or
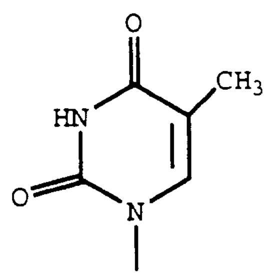

are effective against one or more of the following viruses: herpes simplex virus 1 and 2, varicella-zoster virus, and cytomegalovirus. They are also believed to be active against other DNA viruses. Exemplary DNA viruses in addition to those named above include other herpes viruses (e.g., Epstein-Barr virus, pseudorabies virus, human herpes virus 6, and the like), poxvirus (e.g., vaccinia virus, monkey pox, and myoma), papovaviruses (e.g., the papilloma viruses), hepatitis B virus, and adenoviruses. All of the other compounds of formula 1 are believed to be active against one or more of the following viruses: herpes simplex virus 1 and 2, varicella-zoster virus, cytomegalovirus, and the other DNA viruses described above.

The compounds of this invention may be administered parenterally (for example, by intravenous, intraperitoneal or intramuscular injection), orally or topically.

The compounds may be administered orally or parenterally in an amount effective to treat the infection. The dosage will, of course, depend on the severity of the infection, but will likely be in the range of about 1.0 to 50 mg./kg. of body weight. The desired dose may be administered several times daily at appropriate intervals.

For infections of the eye, or other external tissues, (e.g., mouth and skin), the composition may be applied to the infected part of the body of the patient topically as an ointment, cream, aerosol, gel, powder, lotion, suspension or solution (e.g., as in eye drops). The concentration of the compound in the vehicle will, of course, depend on the severity of the infection, but will likely be in the range of about 0.1 to 7% by weight.

The compounds of this invention can be prepared from a compound of formula

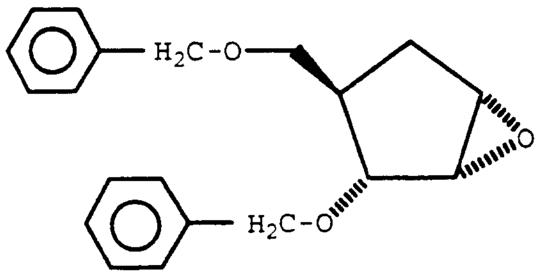

2 .

Protection of the hydroxyl groups in the known compound of formula

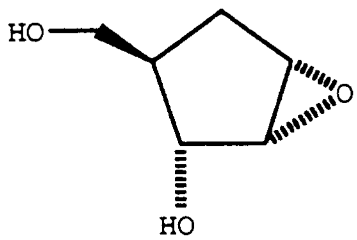

3

using benzyl bromide and sodium hydride affords the compound of formula 2.

The epoxide of formula 3 is described in the literature as note Paulsen et al., Chem. Ber., Vol. 114, p. 346-358 (1981) and Hutchinson et al., J. Heterocyclic Chem., Vol. 26, p 451-452(1989). Using a modification of these procedures, the epoxide of formula 3 can be prepared by reacting the cyclopentadiene of formula

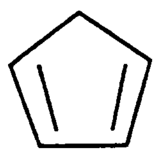

4

with paraformaldehyde in acetic acid with a catalytic amount of para-toluenesulfonic acid, followed by treatment with sodium methoxide in methanol to give the compound of formula

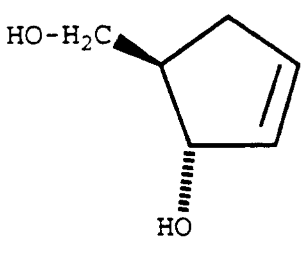

5 .

Treatment of compound 5 with meta-chloroperoxybenzoic acid yields the epoxide of formula 3.

The protected epoxide of formula 2 can alternatively be prepared from the compound of formula

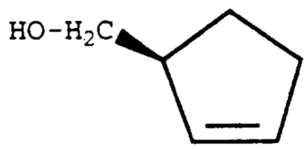

6

by treating with sodium hydride followed by benzyl bromide to give the compound of formula

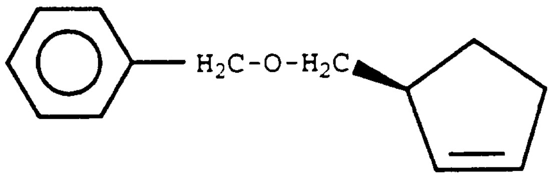

.

7

Reaction of compound 7 with N-bromosuccinimide in aqueous dioxane followed by treatment of the resulting bromohydrin with methanolic potassium carbonate provides the compound of formula

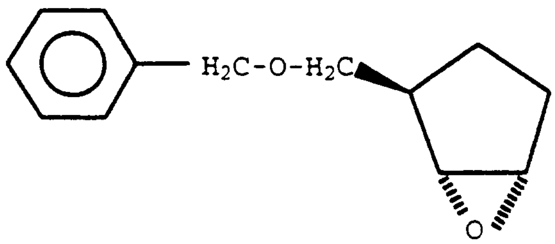

.

8

Treatment of compound 8 with sodium phenylselenide in ethanol followed by reacting the resulting phenylselenide with hydrogen peroxide yield the compound of formula

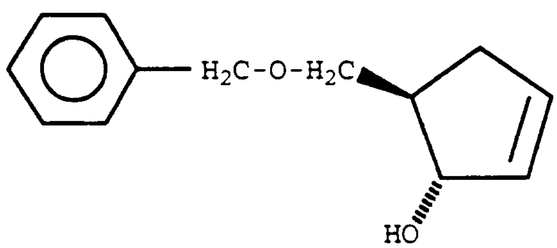

.

9

Epoxidation of compound 9 with meta-chloroperoxybenzoic acid in buffered dichloromethane provides the compound of formula

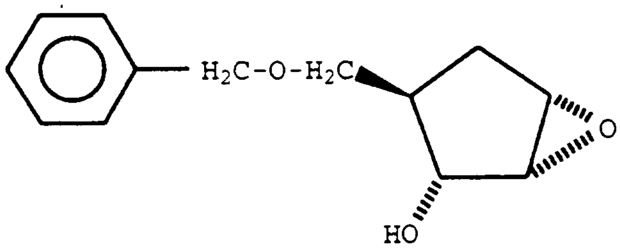

10

Treatment of compound 10 with sodium hydride followed by benzyl bromide yields the protected epoxide of formula 2. The compound of formula 6 is described in the literature as note Branner-Jorgensen et al., Acta Chem. Scand., Vol. 20, p 2192 - 2194 (1966); Maercker et al., Chem. Ber., Vol. 106, p. 773 - 797 (1973); and Chapman et al., J. Amer. Chem Soc., Vol. 100, p. 4878 - 4884 (1978).

A compound of formula 1 wherein $R_1$ is

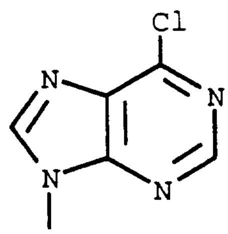

and $R_6$ and $R_7$ are hydrogen can be prepared by reaction of a compound of formula 2 with a compound of formula

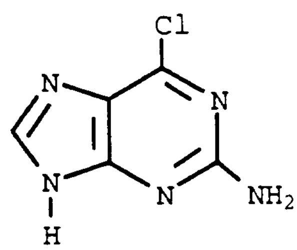

11

in the presence of a base such as lithium hydride, sodium hydride, or potassium hydride in an aprotic polar solvent such as dimethylformamide, dimethyl sulfoxide, or sulfolane (tetramethylene sulfone). This yields the corresponding compound of the formula

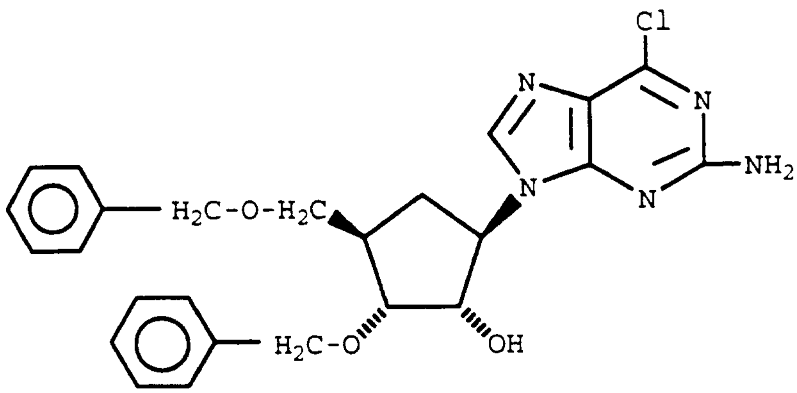

.

12

Protection of the amino (-$NH_2$) group in the compound of formula 12 affords a compound of formula

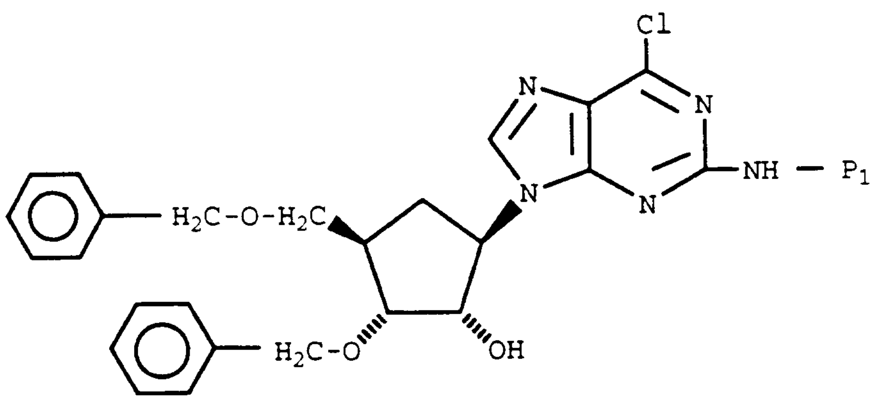

13

wherein the protecting group $P_1$ can be trityl or substuted trityl (e.g. 4-monomethoxytrityl or 4,4'-dimethoxytrityl). The protection can be accomplished by treatment of the compound of formula 12 with trityl chloride or a substituted trityl chloride in dichloromethane in the presence of triethylamine (and, optionally, in the presence of 4-N,N-dimethylaminopyridine). Oxidation of the hydroxyl group in this compound of formula 13 yields a compound of formula

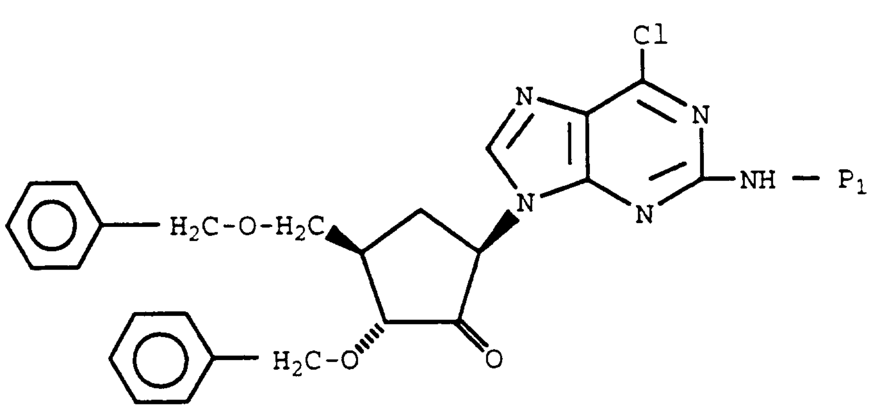

.

14

The oxidation can be carried out by methods well known in the art (e.g. oxalyl chloride/dimethyl sulfoxide/triethylamine in dichloromethane or 1,1,1-triacetoxy-1,1-dihydro-1,2-benziodoxol-3(1H)-one and

tert-butanol in dichloromethane). Treatment of a compound of formula 14 with a methylenation reagent such as zinc/titanium tetrachloride/dibromomethane in dichloromethane/tetrahydrofuran or methylenetriphenylphosphorane yields a compound of formula

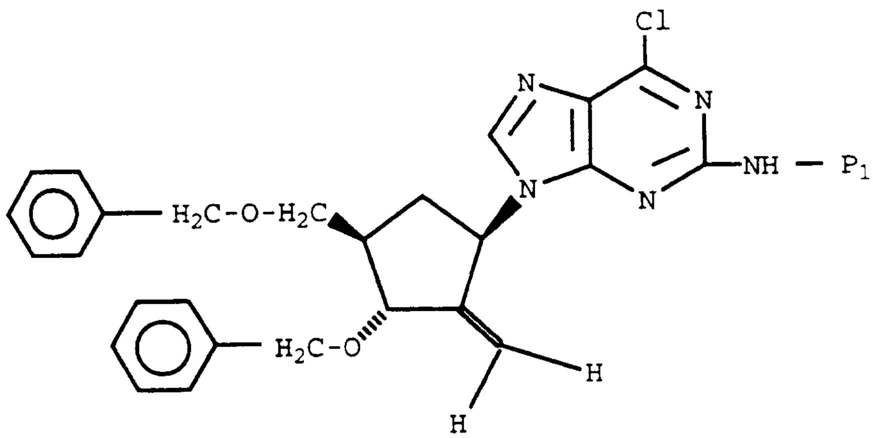

.

15

Removal of the protecting groups from a compound of formula 15 provides the compound of formula 1 wherein $R_1$ is

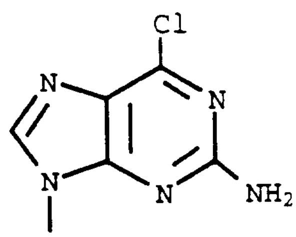

and $R_6$ and $R_7$ are hydrogen. Removal of the protecting group $P_1$ can be accomplished by treatment with aqueous alcoholic mineral acid (e.g. hydrochloric acid in aqueous methanol) or aqueous acetic acid. Subsequently, the benzyl protecting groups can be removed by treatment with boron trichloride. Alternatively, the $P_1$ and benzyl protecting groups can all be removed by treatment with boron trichloride.

The compound of formula 1 wherein $R_1$ is

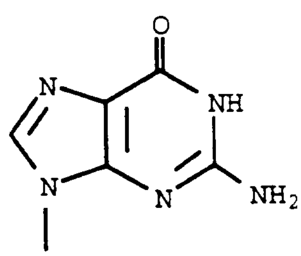

and $R_6$ and $R_7$ are hydrogen can be prepared as follows:

Reaction of a compound of formula 2 with a compound of formula

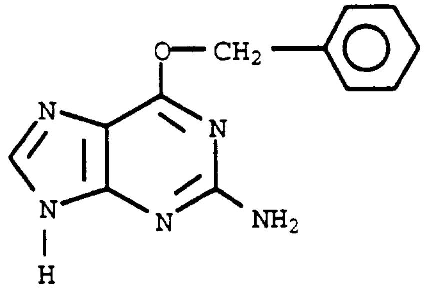

16

according to procedures analogous to those used in preparation of the compound of formula 12 affords a compound of formula

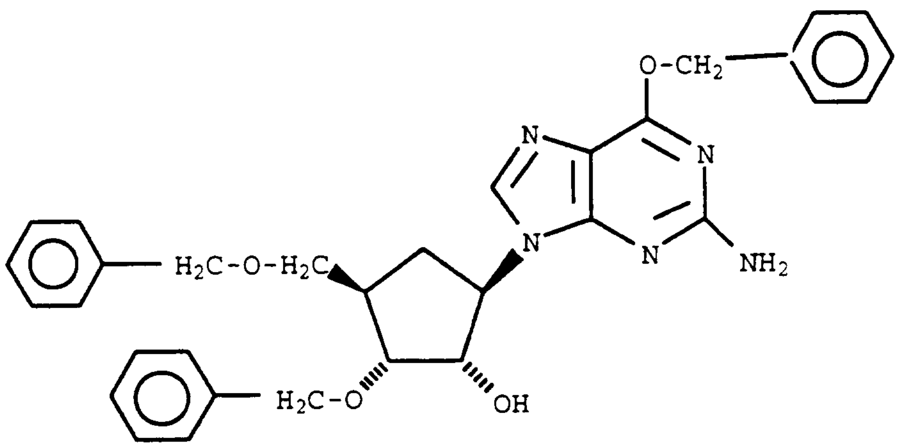

17

Protection of the amino group ($-NH_2$) in the compound of formula 17 according to the procedures analogous to those used in the preparation of the compound of formula 13 yields a compound of the formula

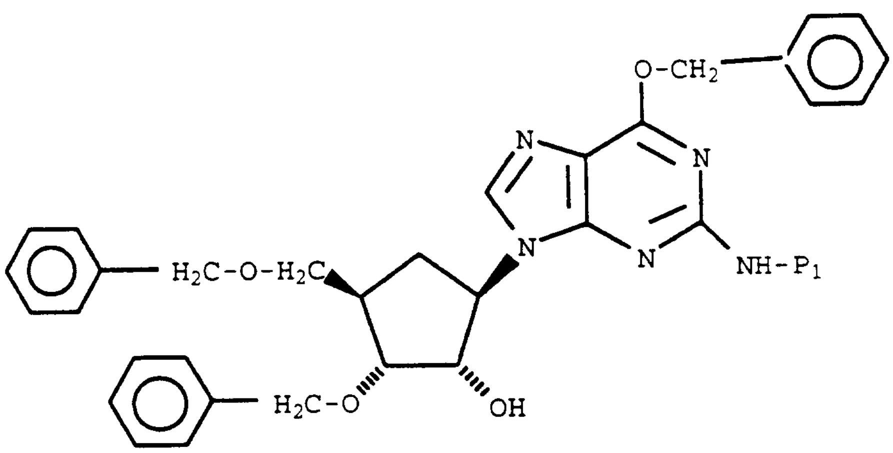

18

Oxidation of the alcohol group in the compound of formula 18 under conditions analogous to those used in the preparation of the compound of formula 14 provides a compound of formula

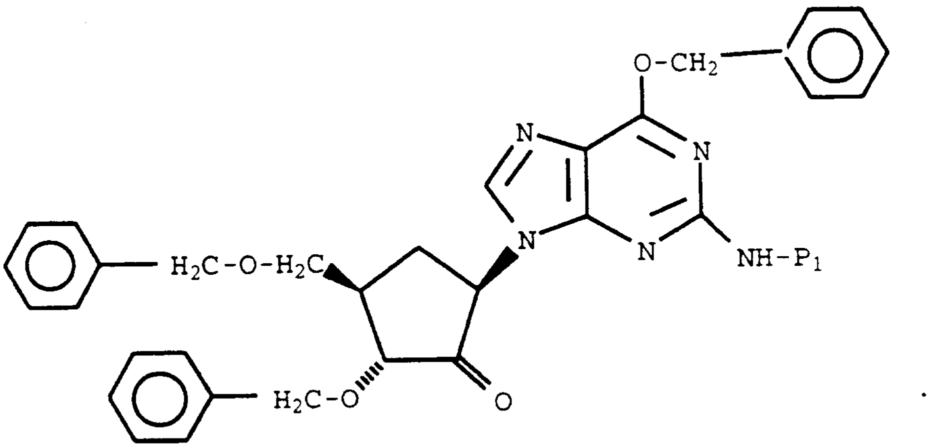

19

Methylenation of the ketone group in the compound of formula 19 with zinc/titanium tetrachloride/dibromomethane in dichloromethane yields a compound of formula

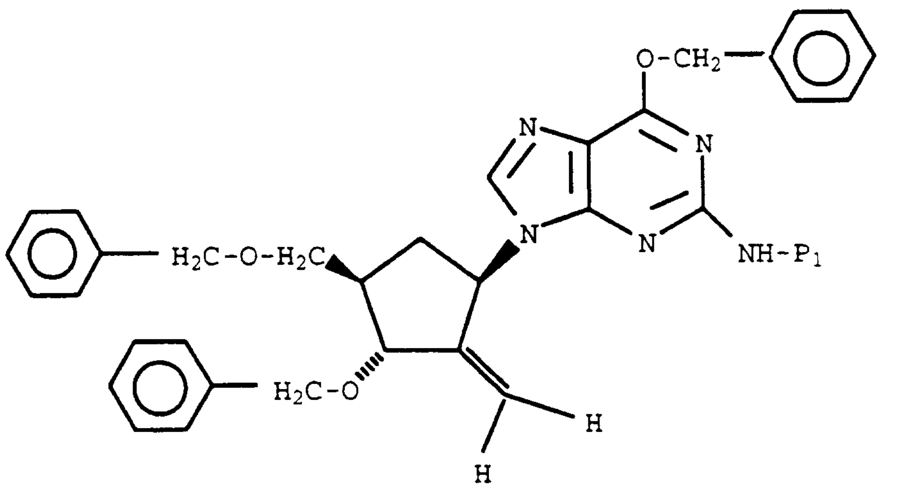

20

Finally, removal of the protecting groups from the compound of formula 20 provides the compound of formula 1 wherein $R_1$ is

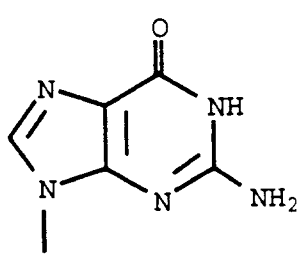

and $R_6$ and $R_7$ are hydrogen. Removal of the $P_1$ and the purine O-benzyl protecting groups can be accomplished by treatment with aqueous alcoholic mineral acid (e.g., hydrochloric acid in aqueous methanol). Subsequently, the hydroxyl benzyl protecting groups can be removed by treatment with boron trichloride. Alternatively, all the protecting groups can bne removed by treatment with boron trichloride.

Alternatively, this compound can be prepared from a compound of formula 1 wherein $R_1$ is

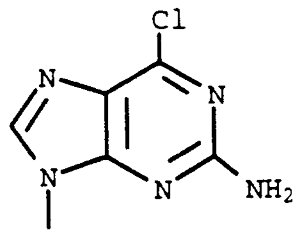

and $R_6$ and $R_7$ are hydrogen, by hydrolysis of the chloro group using aqueous acid (e.g. aqueous hydrochloric acid).

The compound of formula 1 wherein $R_1$ is

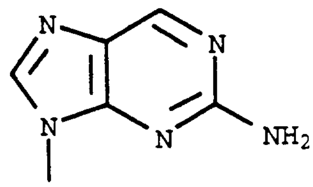

and $R_6$ and $R_7$ are hydrogen can be prepared from the compound of formula 12. Hydrogenolysis of this compound (e.g. ammonium formate and palladium on carbon in methanol; palladium hydroxide on carbon and cyclohexene in ethanol; or palladium on carbon, hydrogen, and ethanol) results in reduction of the chloro group and removal of the benzyl protecting groups to afford the compound of formula

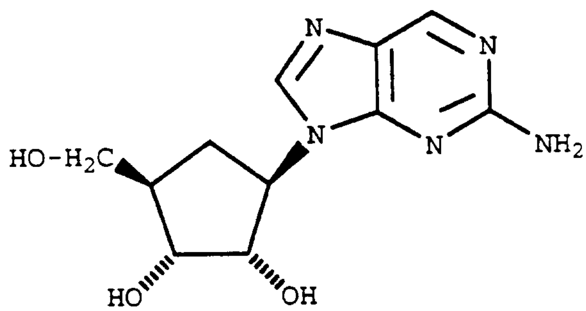

21

Reaction of the compound of formula 21 with 1,3-dichloro-1,1,3,3-tetraisopropyldisiloxane in pyridine affords the compound of formula

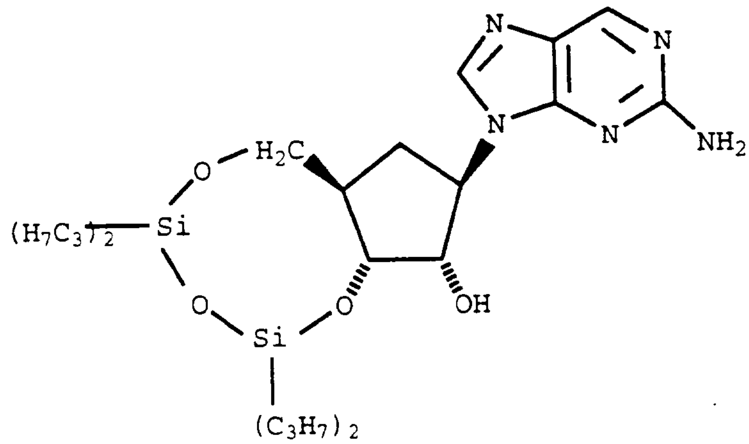

22

Protection of the amino (-$NH_2$) group in the compound of formula 22 by reacting with trityl chloride or a substituted trityl chloride in the presence of triethylamiine and, optionally, 4-N,N-dimethylaminopyridine, affords the compound of the formula

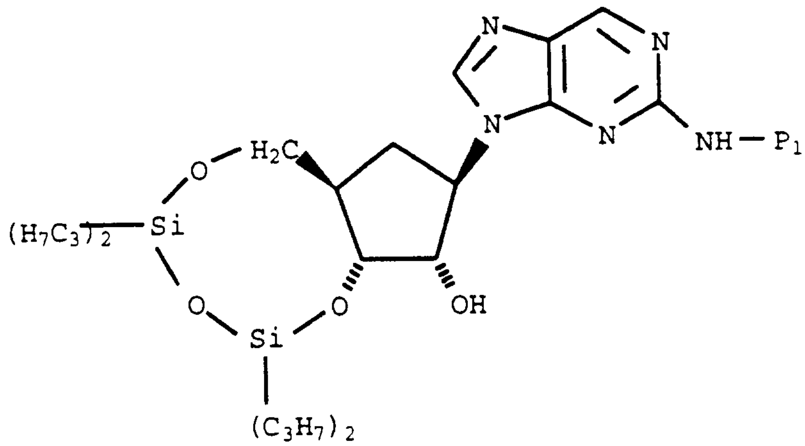

23

where $P_1$ is trityl or substituted trityl. Sequential oxidation and methylation of the compound of formula 23 under conditions analogous to those used in the preparation of the compounds of formulas 14 and 15, respectively, followed by removal of the protecting groups, affords the compounds of formula 1 wherein $R_1$ is

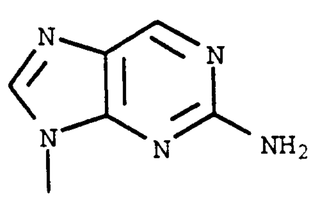

,

and $R_6$ and $R_7$ are hydrogen. The silyl protecting group can be removed first by treatment with fluoride ion (e.g. tetrabutylammonium fluoride in tetrahydrofuran), and then the trityl protecting group $P_1$ can be removed by treatment with aqueous alcoholic mineral acid (e.g. hydrochloric acid in aqueous methanol) or aqueous acetic acid. Alternatively, the trityl protecting group $P_1$ can be removed first followed by removal of the silyl protecting group.

Alternatively, reaction of the compound of formula

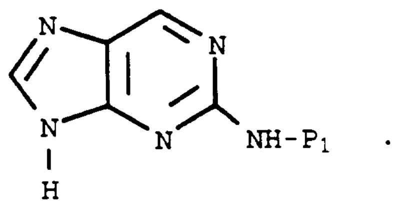

24

wherein $P_1$ is a trityl or substituted trityl group, with a compound of formula 2 under conditions analogous to those used in the preparation of the compound of formula 12 affords a compound of formula

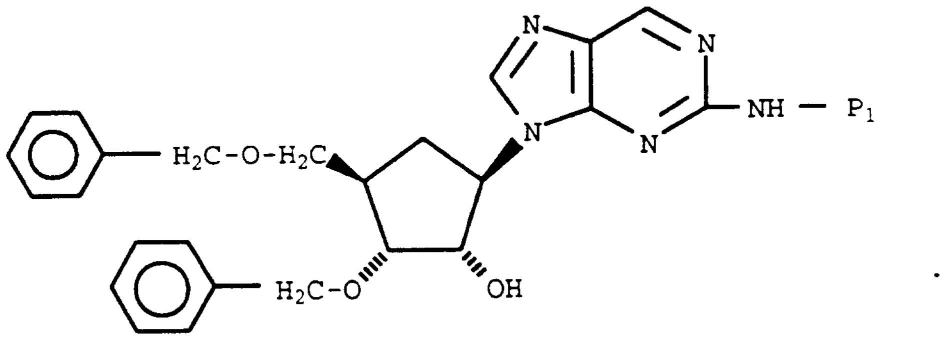

25

Sequential oxidation and methylenation of the compound of formula 25 under conditions analogous to those used in the preparation of the compounds 14 and 15, respectively, followed by removal of the protecting groups affords the compound of formula 1 wherein $R_1$ is

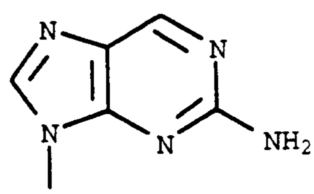

and $R_6$ and $R_7$ are hydrogen.

Alternatively, reaction of a compound of formula

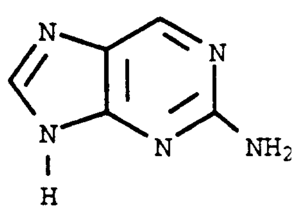

26

with a compound of formula 2 affords a compound of formula

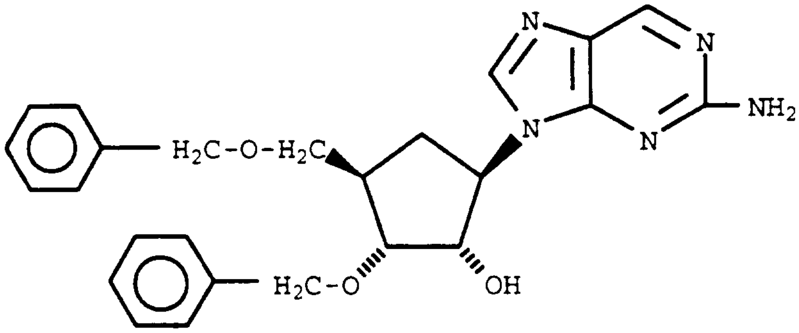

27

Protection of the amino ($-NH_2$) group with a trityl or substituted trityl group provides the compound of formula 25, which can then be converted (as described above) to the compound of formula 1 wherein $R_1$ is

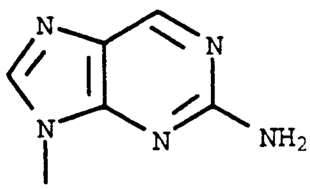

and $R_6$ and $R_7$ are hydrogen.

The compound of formula 1 wherein $R_1$ is

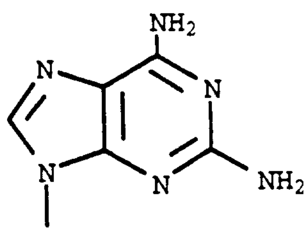

and $R_6$ and $R_7$ are hydrogen can be prepared from a compound of formula 15 by methods known in the art. Thus, for example, when a compound of formula 15 wherein $P_1$ is trityl is treated with hot methanolic ammonia, displacement of the chloro group with an amino group will result. The protecting groups can then be removed according to procedures known in the art.

Alternatively, this compound of formula 1 can be prepared from a compound of formula 1 wherein $R_1$ is

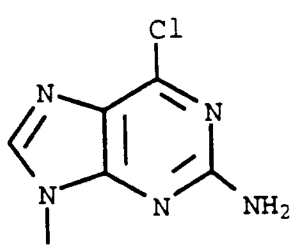

and $R_6$ and $R_7$ are hydrogen by methods known in the art. [See, e.g., J.C. Martin, et al., J.Med. Chem. Vol. 28, 358 (1985)].

A compound of formula 1 wherein $R_1$ is

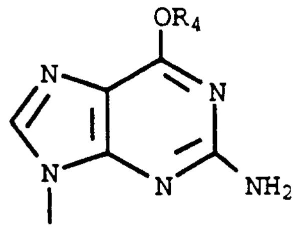

and $R_6$ and $R_7$ are hydrogen can be prepared by treatment of a compound of formula 12 with sodium alkoxide, which provides the compound of formula

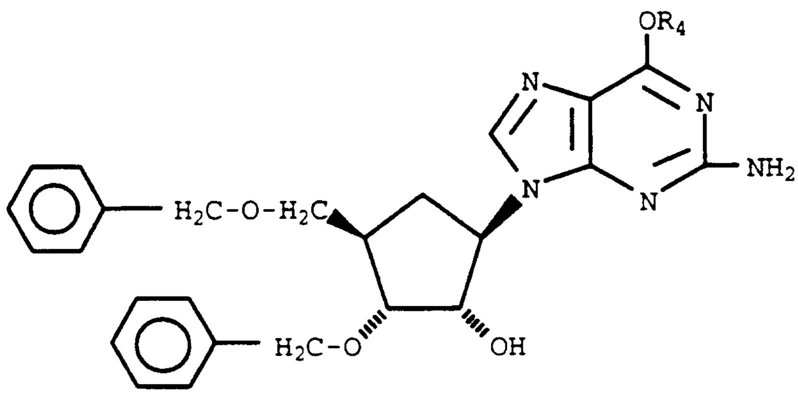

.

28

Protection of the amino group, oxidation and methylenation (according to procedures analogous to those used in the preparation of compounds 13, 14 and 15), followed by removal of protecting groups, provides the compound of formula 1 wherein $R_1$ is

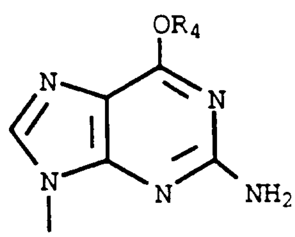

and $R_6$ and $R_7$ are hydrogen.

Alternatively, this compound of formula 1 can be prepared from a compound of formula 1 wherein $R_1$ is

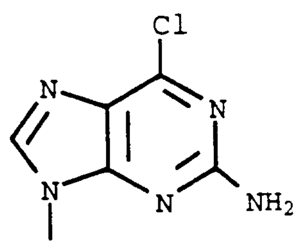

and $R_6$ and $R_7$ are hydrogen by methods known in the art. See, for example, J.F. Gerster, et al., J. Amer. Chem. Soc., 87, 3752 (1965); K.K. Ogilvie, et al., Can. J. Chem., 62, 2702 (1984); M.R. Harnden, et al., J. Med. Chem., 30, 1636 (1987).

Alternatively, this compound of formula 1 can be prepared from a compound of formula

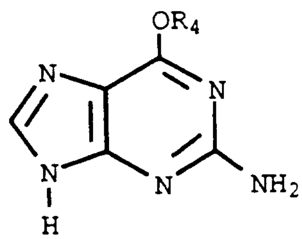

29

and a compound of formula 2 using procedures analogous to those used in the preparation of compounds 12, 13, 14, and 15, followed by removal of the protecting groups. A compound of formula 29 can be prepared from the compound of formula 11 by methods known in the art [See, e.g., W.A. Bowles, et al., J. Med. Chem., 6, 471 (1963); M. MacCoss, et al., Tetrahedron Lett., 26, 1815 (1985)].

The compound of formula 1 wherein $R_1$ is

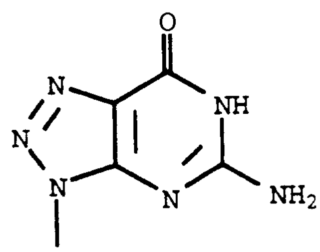

and $R_6$ and $R_7$ are hydrogen can be prepared from a compound of formula 2 and a compound of formula

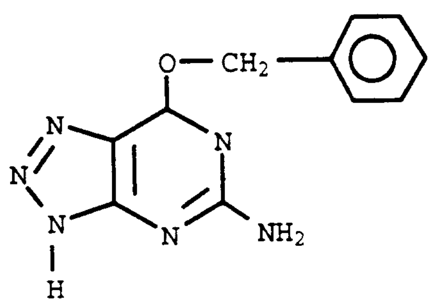

30

according to procedures analogous to those used in preparation of compounds 17, 18, 19, and 20, followed by removal of the protecting groups.

The compounds of formula 1 wherein $R_1$ is

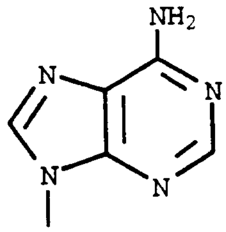

and $R_6$ and $R_7$ are hydrogen can be prepared by the reaction of the compound of formula

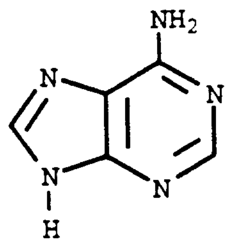

31

with a compound of formula 2 according to procedures analogous to those used in preparation of the compound of formula 12, affording a compound of formula

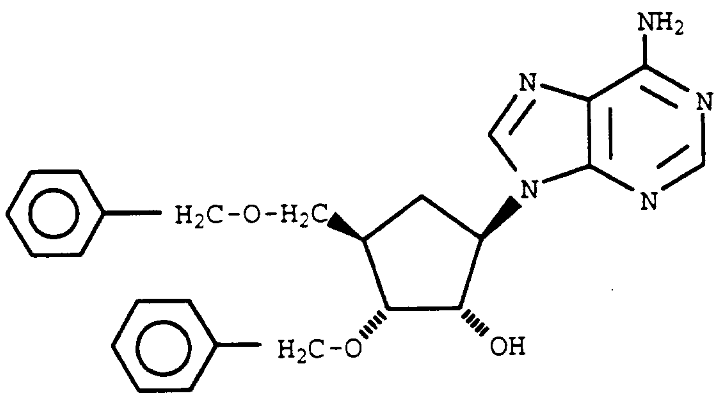

32

Selective protection of the amino ($-NH_2$) group in the compound of formula 32 with an acyl group $P_2$ (e.g., acetyl) gives the compound of formula

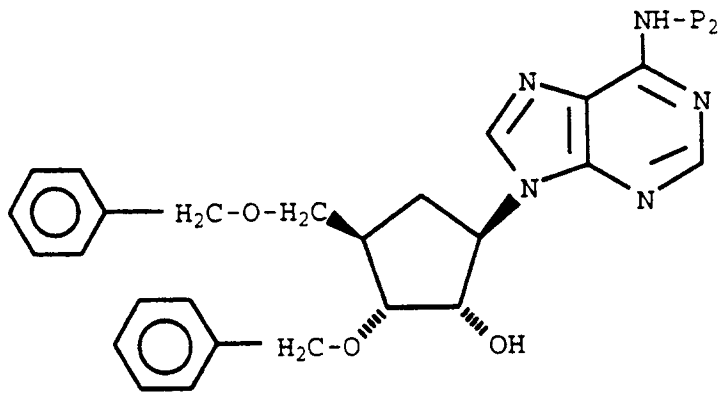

.

33

[See, for example, G.S. Ti et al., J. Amer. Chem. Soc., 104, 1316(1982)]. Oxidation of the compound of formula 33 and subsequent treatment with zinc/titanium tetrachloride/dibromomethane or methylenetriphenylphosphorane according to procedures analogous to those used in the preparation of compounds 14 and 15 provides a compound of the formula

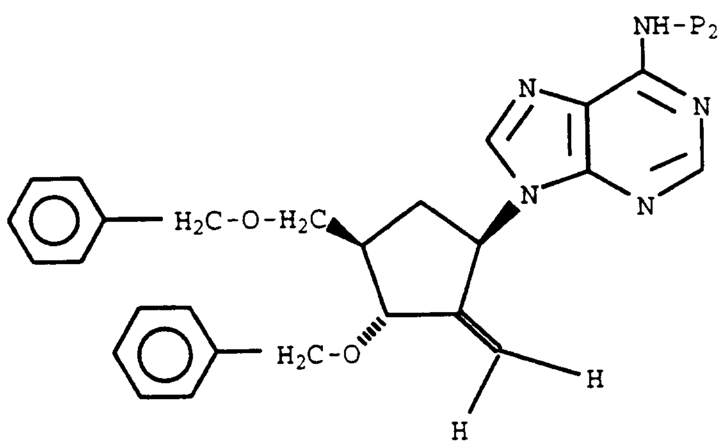

.

34

Removal of the protecting groups from this compound yields the compound of formula 1 wherein $R_1$ is

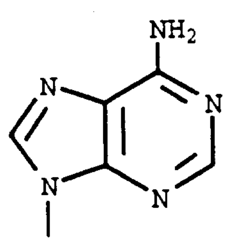

and $R_6$ and $R_7$ are hydrogen. The amino protecting group $-P_2$ can be removed by treating with sodium methoxide in methanol or methanolic ammonia.

Alternatively, this compound of formula 1 can be prepared by reaction of the compound of formula

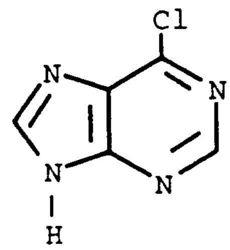

35

with a compound of formula 2 under conditions analogous to those used for the preparation of the compound of formula 12. Subsequent oxidation and methylenation according to procedures analogous to those used in the preparation of compounds 14 and 15 provides the compound of formula

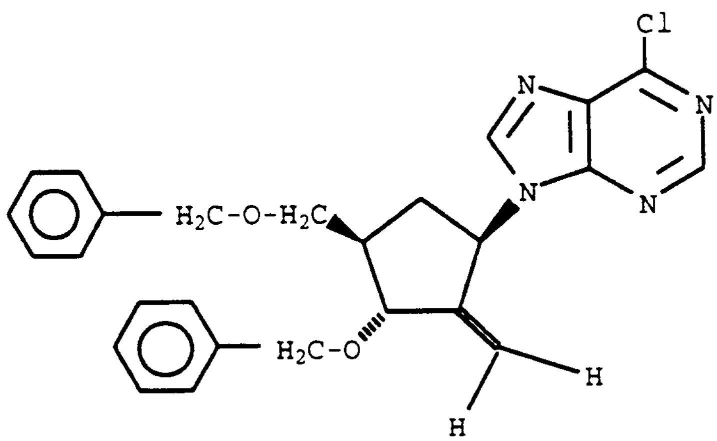

36

Treatment of a compound of formula 36 with hot ammonia in an alcohol (such as methanol or ethanol) followed by removal of protecting groups yields the compound of formula 1 wherein $R_1$ is

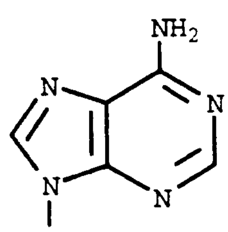

and $R_6$ and $R_7$ are hydrogen.

In another alternative, this compound of formula 1 can be prepared from a compound of formula 1 wherein $R_1$ is

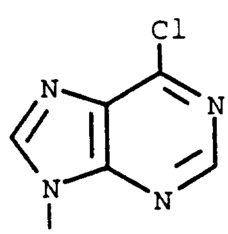

and $R_6$ and $R_7$ are hydrogen by methods known in the art. [See e.g., J. C. Martin et al., J. Med. Chem., 28, 358 (1985)]

The compound of formula 1 wherein $R_1$ is

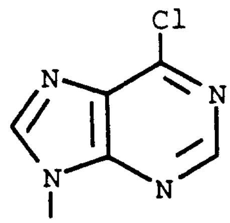

and $R_6$ and $R_7$ are hydrogen can be prepared by removal of the protecting groups from the compound of formula 36 using boron trichloride.

The compound of formula 1 wherein $R_1$ is

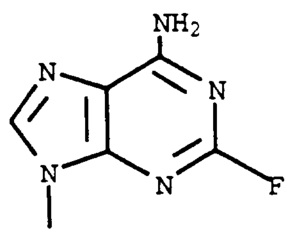

and $R_6$ and $R_7$ are hydrogen can be prepared from the compound of formula 1 wherein $R_1$ is

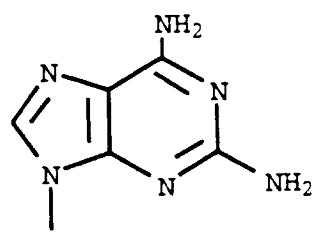

and $R_6$ and $R_7$ are hydrogen by following known procedures. See, for example, J.A. Montgomery et al., "Synthesic Procedures in Nucleic Acid Chemistry", Vol. 1, W.W. Zorbach and R.S. Tipson, Eds., Interscience Publishers (John Wiley and Sons), N.Y. p. 205, 1968.

The compound of formula 1 wherein $R_1$ is

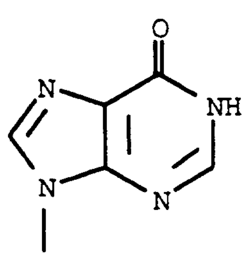

and $R_6$ and $R_7$ are hydrogen can be prepared from this compound of formula 1 wherein $R_1$ is

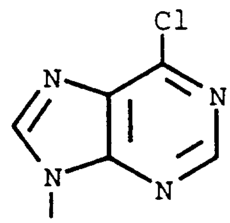

and $R_6$ and $R_7$ are hydrogen by acid hydrolysis (e.g. hot aqueous hydrochloric acid) or basic hydrolysis (e.g., aqueous methanolic sodium hydroxide).

Alternatively, this compound of formula 1 can be prepared by treatment of a compound of formula 1 wherein $R_1$ is

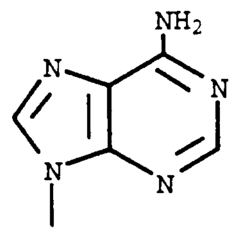

and $R_6$ and $R_7$ are hydrogen with adenosine deaminase of nitrous acid according to methods known in the art [e.g., M. J. Robins, et al., J. Med. Chem., 27, 1486 (1984); K.K. Ogilvie et al., Can. J. Chem., 62 241 (1984)]; I. Iwai, et al., in "Synthetic Procedures in Nucleic Acid Chemistry", Vol. 1, W.W. Zorbach and R.S. Tipson, Eds., Interscience Publishers (John Wiley and Sons), N.Y., p. 135, 1968; R.E. Holmes et al., J. Amer. Chem. Soc., 86, 1242 (1964).

The compound of formula

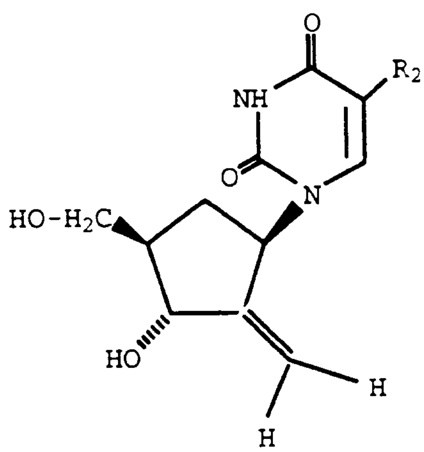

37

wherein $R_2$ is hydrogen, fluoro, methyl, ethyl, n-propyl, 2-chloroethyl, or 2-fluoroethyl can be prepared by reaction of the corresponding compound of formula

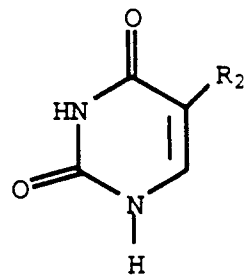

38

with a compound of formula 2 in the presence of a base such as lithium hydride, sodium hydride, or potassium hydride in an aprotic polar solvent such as dimethylformamide, dimethyl sulfoxide or sulfolane to yield a compound of formula

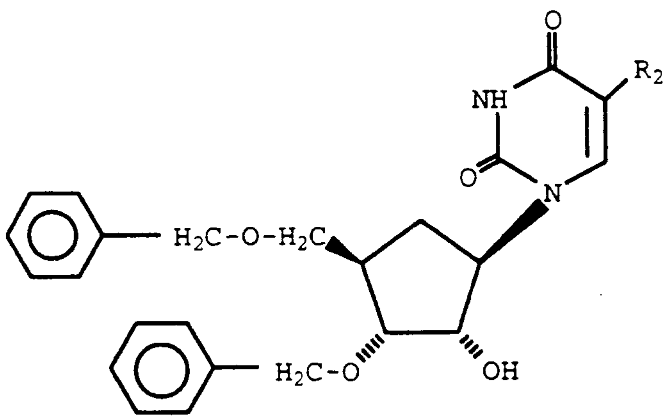

39

The compound of formula 39 can also be prepared by reaction of a mono-tetra-alkylammonium salt (e.g. tetra-butylammonium salt) of the compound of formula 38 with a compound of formula 2 in an aprotic polar solvent such as dimethylformamide, dimethylsulfoxide, or sulfolane. The mono-tetra-alkylammonium salt of the compound of formula 38 can be prepared by reaction of a compound of formula 38 with an aqueous solution of a tetra-alkylammonium hydroxide (e.g., tetra-butylammonium hydroxide) in an aprotic polar solvent such as dimethylformamide.

Oxidation of the hydroxyl group in a compound of formula 39 using known methods in the art (e.g., oxalyl chloride/dimethylsulfoxide/triethylamine in dichloromethane or 1,1,1-triacetoxy-1,1-dihydro-1,2-benziodoxol-3(1H)- one and tert-butanol in dichloromethane) provides the compound of formula

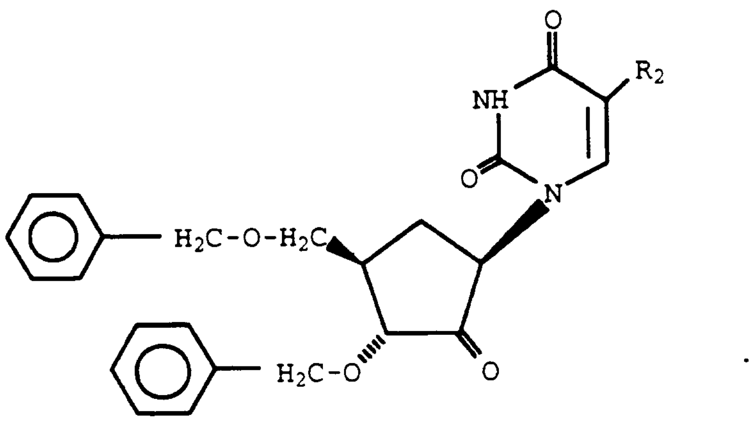

40

Treatment of the compound of formula 40 with a methylenation reagent such as zinc/titanium tetrachloride/dibromomethane in dichloromethane/tetrahydrofuran or methylenetriphenylphosphorane yields the compound of formula

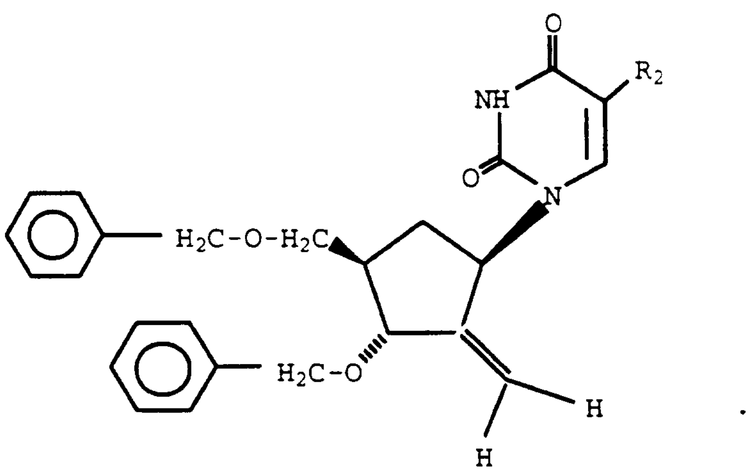

41

Removal of the protecting groups from the compound of formula 41 provides the corresponding compound of formula 37. The benzyl groups can be removed by treatment with boron trichloride.

The compound of formula 38 wherein $R_2$ is 2-chloroethyl or 2-fluoroethyl can be prepared by methods known in the art [H. Griengl et al., J. Med., 30, 1199 (1987); J. Med. Chem., 28, 1679 (1985)].

The compound of formula 39 wherein $R_2$ is fluoro can also be prepared from the corresponding compound 39 wherein $R_2$ is hydrogen by fluorination using trifluoromethyl hypofluorite following methodology known in the art. For example, see M. J. Robins, et al., J. Amer. Chem. Soc., 93, 5277 (1971); Chem. Communs., 18 (1972); T. S Lin et al., J. Med. Chem., 26, 1691 (1983).

The compounds of formula 37 wherein $R_2$ is 2-chloroethyl and 2-fluoroethyl can also be prepared from a compound of formula

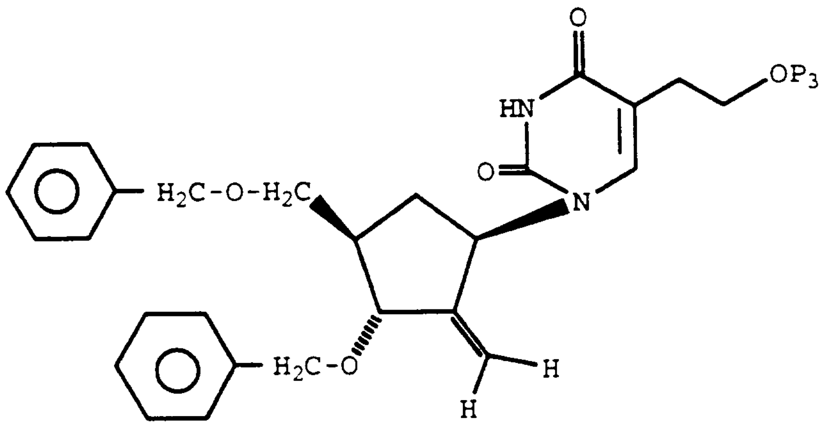

42

wherein $P_3$ is trityl, substituted trityl, or a silyl protecting group. Removal of the $P_3$ protecting group yields a compound of formula 41 wherein $R_2$ is 2-hydroxyethyl. Treatment of this compound with triphenylphosphine/carbon tetrachloride, and subsequent removal of the benzyl protecting groups with boron trichloride, affords the compound of formula 37 wherein $R_2$ is 2-chloroethyl. Similar treatment using triphenylphosphine/N-bromosuccinimide or triphenylphosphine/N-bromosuccinamide/tetrabutyl-ammonium iodide in place of triphenylphosphine/carbon tetrachloride [e.g., see H. Griengl, et al., J. Med Chem., 28, 1679 (1985)] affords compounds of formula 41 wherein P is benzyl and $R_2$ is 2-bromoethyl or 2-iodoethyl, respectively. Subsequent treatment with fluoride ion, followed by removal of the benzyl protecting groups provides the compound of formula 37 wherein $R_2$ is 2-fluoroethyl. Alternatively, treatment of a compound of formula 41 wherein $R_2$ is 2-hydroxyethyl with diethylaminosulfur trifluoride provides, upon removal of the benzyl protecting groups, a compound of formula 37 wherein $R_2$ is 2-fluoroethyl.

The compound of formula 42 can be prepared from a compound of formula

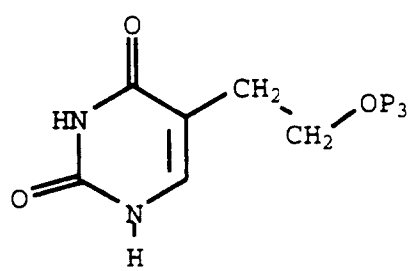

43

and a compound of formula 2 by methods analogous to those used in the preparation of compounds 39, 40 and 41 wherein $R_2$, for example, is hydrogen, methyl or ethyl. The compounds of formula 43 can be prepared from the corresponding free alcohol by methods known in the art.

The compound of formula

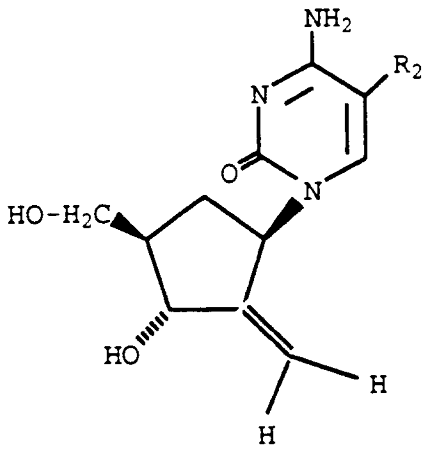

44

wherein $R_2$ is hydrogen, fluoro, methyl, ethyl, n-propyl, 2-chloroethyl or 2-fluoroethyl can be prepared from the corresponding compound of formula 41. Treatment of this compound with, for example, 4-chlorophenyl dichlorophosphate and 1,2,4-triazole in a solvent such as pyridine and reaction of the resulting intermediate with ammonium hydroxide in a solvent such as dioxane provides the corresponding compound of formula

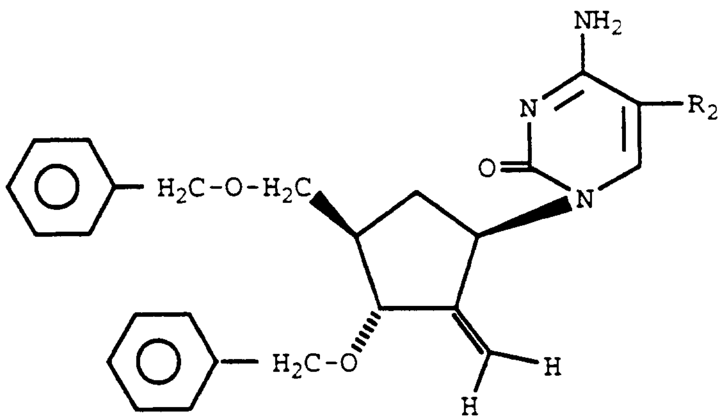

45

See, for example, T.S. Lin et al., J. Med. Chem., 26, 1691(1983); P. Herdewijn, et al., J. Med. Chem., 28, 550 (1985). Removal of the protecting groups from the compound of formula 45 yields the correspopnding compound of formula 44. The benzyl protecting groups can be removed by treatment with boron trichloride. Alternatively, the compound of formula 41 wherein $R_2$ is hydrogen, fluoro, methyl, ethyl, n-propyl, 2-chloroethyl or 2-fluoroethyl can be reacted with a sulfonyl chloride (e.g., p-toluenesulfonyl chloride) in an inert solvent (e.g., 1,2-dichloroethane or dioxane) in the presence of a base such as potassium carbonate. (For other sulfonyl chlorides and solvents, see, for example, European Patent Application EP 204,264). This affords the corresponding compound of formula

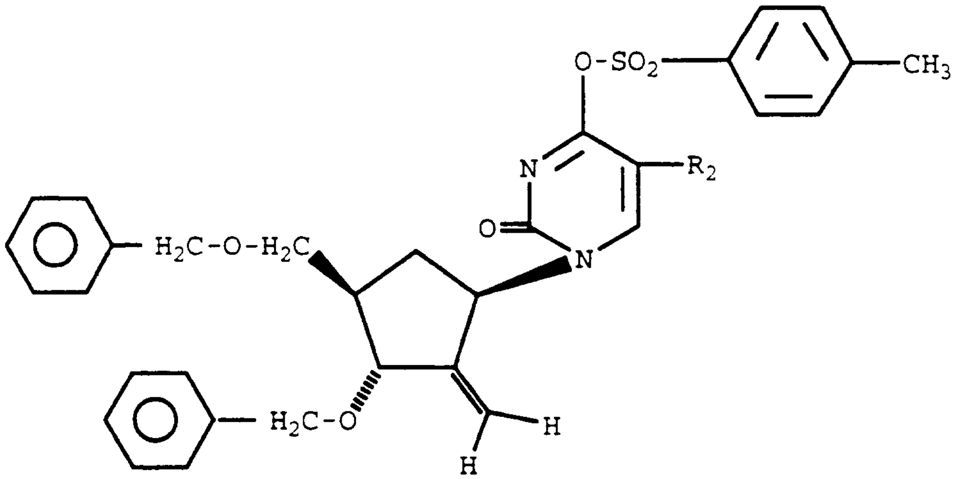

46

which can be treated with ammonia gas in an inert solvent (e.g., 1,2-dichloroethane or dioxane) to afford the corresponding compound of formula 45. Removal of the protecting groups from the compound of formula 45 yields the corresponding compound of formula 44.

Alternatively, the compound of formula 44 wherein $R_2$ is fluoro, hydrogen, methyl, ethyl, n-propyl, 2-chloroethyl or 2-fluoroethyl can be prepared from the corresponding compound of formula 37 by methods known in the art. See, for example, T. S. Lin et al., J. Med. Chem., 26, 1691(1983); P. Herdewijn, et al., J. Med. Chem., 28, 550 (1985); European Patent Application EP 360,018.

Alternatively, the compound of formula 44 wherein $R_2$ is fluoro, hydrogen, methyl, ethyl, n-propyl, 2-chloroethyl or 2-fluoroethyl can be prepared by reaction of the corresponding compound of formula

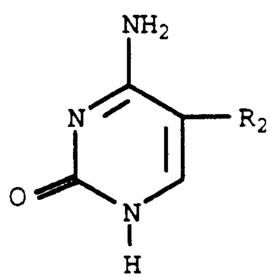

47

with a compound of formula 2 in the presence of lithium hydride, sodium hydride or potassium hydride in an aprotic polar solvent such as dimethylformamide, dimethyl sulfoxide or sulfolane to yield a compound of formula

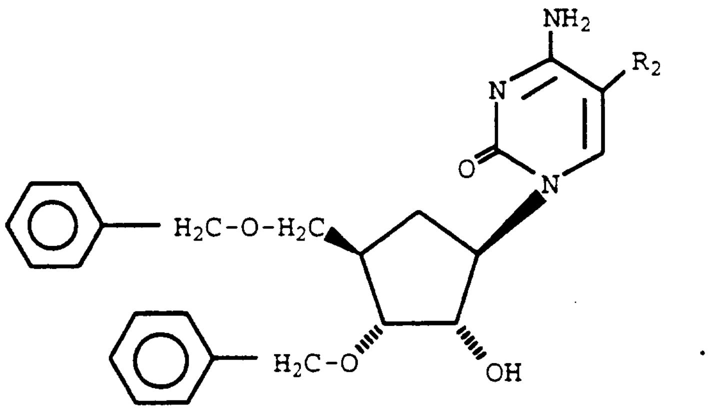

48

Selective protection of the amino group in the compound of formula 48 with an acyl group $R_2$ (e.g., acetyl) yields a compound of formula

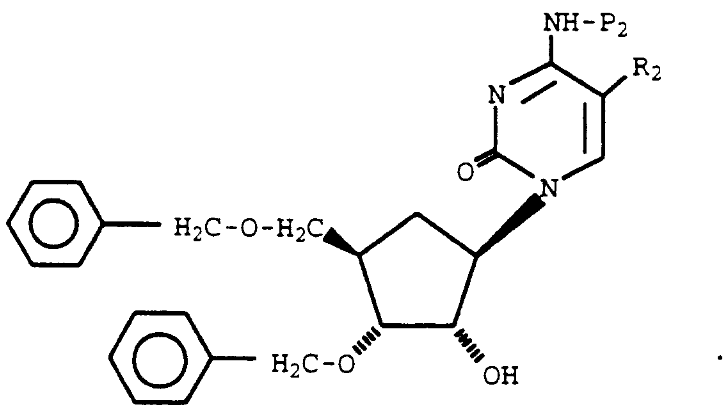

49

[See, for example, G.S. Ti et al., J. Amer. Chem. Soc., 104, 1316 (1982)]. Oxidation of the compound of formula 49, followed by methylenation with zinc/titanium tetrachloride/dibromomethane, with subsequent or simultaneous removal of the acyl protecting group $P_2$, gives a compound of formula 45. Removal of the protecting groups from this compound provides a compound of formula 44 wherein $R_2$ is fluoro, hydrogen, methyl, ethyl, n-propyl, 2-chloroethyl or 2-fluoroethyl. The benzyl groups can be removed by treatment with boron trichloride in dichloromethane.

Alternatively, oxidation of the compound of formula 49 and subsequent treatment with methylenetriphenylphosphorane gives a compound of formula

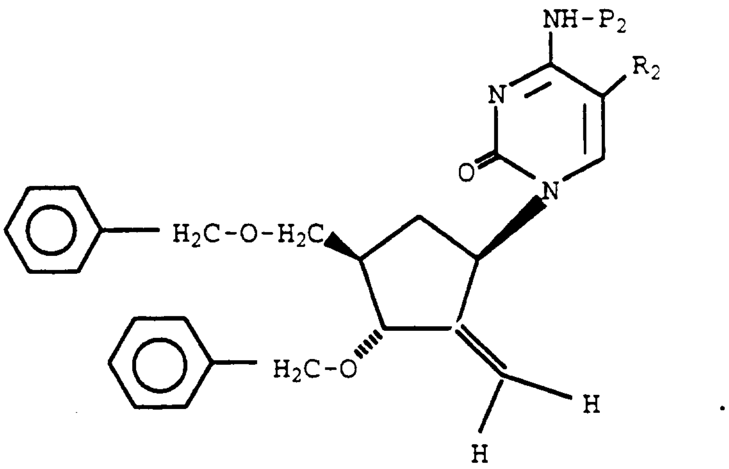

50

Removal of the protecting groups from this compound provides the compound of formula 44 wherein $R_2$ is fluoro, hydrogen, methyl, ethyl, n-propyl, 2-chloroethyl or 2-fluoroethyl. Removal of the acyl protecting group $P_2$ can be accomplished using sodium methoxide in methanol or methanolic ammonia.

Alternatively, the compound of formula 48 wherein $R_2$ is hydrogen, fluoro, methyl, ethyl, n-propyl, 2-chloroethyl or 2-fluoroethyl can be prepared from the corresponding compound of formula 39 using methodology known in the art. See, for example, T. S. Lin et al., J. Med. Chem., 26, 1691(1983); P. Herdewijn, et al., J. Med. Chem., 28, 550 (1985); European Patent Application EP 360018 and EP 204264.

Alternatively, the compound of formula 48 or 49 wherein $R_2$ is fluoro can be prepared from the corresponding compound of formula 48 or 49 wherein $R_2$ is hydrogen by fluorination using trifluoromethyl hypofluorite following methodology known in the art. For example, see M.J. Robins, et al., J. Amer. Chem. Soc., 93, 5277 (1971) and Chem. Communs., 18 (1972); T. S. Lin et al., J. Med. Chem., 26, 1691(1983).

The compound of formula 37 wherein $R_2$ is chloro, bromo or iodo can be prepared from the compound of formula 39 wherein $R_2$ is hydrogen. Halogenation of this compound by methods known in the art provides the corresponding compound of formula 39 wherein $R_2$ is chloro, bromo or iodo. (See, for example, P. Herdwijn et al., J. Med. Chem., 28, 550 (1985); M.J. Robins et al., J., Org. Chem., 48, 1854 (1983); T.S. Lin et al., J. Med. Chem., 26, 598 (1983); T. Ueda et al., Nucleotides and Nucleosides, 4, 401 (1985); "Synthetic Procedures in Nucleic Acid Chemistry", Vol. 1, W.W. Zorback and R. S. Tipson, Eds., John Wiley and Sons, NY, p. 491, (1968). Oxidation of the compound of formula 39 wherein $R_2$ is chloro, bromo, or iodo and subsequent methylenation with zinc/titanium tetrachloride/dibromomethane or methylenetriphenylphosphorane provides the corresponding compound of formula 41 wherein $R_2$ is chloro, bromo or iodo. (See, for example, European Patent Application EP 360,018). Removal of the benzyl protecting groups by treatment with boron trichloride affords the compound of formula 37 wherein $R_2$ is chloro, bromo, or iodo.

The compound of formula 44 wherein $R_2$ is chloro, bromo or iodo can be prepared from the corresponding compound of formula 41 using methods known in the art and analogous to those used in the conversion of compound 41 to compound 44, wherein, for example, $R_2$ is hydrogen, methyl or ethyl.

Alternatively, the compound of formula 44 wherein $R_2$ is chloro, bromo or iodo can be prepared from the corresponding compound of formula 37 by methods known in the art. See, for example, T. S. Lin et al., J. Med. Chem., 26, 1691(1983); P. Herdewijn, et al., J. Med. Chem., 28, 550 (1985); European Patent Applications EP 360018 and EP 204264).

The compound of formula 37 wherein $R_2$ is trifluoromethyl can be prepared from the compound of formula 41 wherein $R_2$ is iodo by treatment with trifluoromethyl iodide and copper followed by the removal of the benzyl protecting groups using boron trichloride. See, for example, Y. Kobayashi et al., J. Chem. Soc. Perkin 1, 2755 (1980); T. S. Lin et al., J. Med. Chem., 26, 1691(1983).

The compound of formula 37 wherein $R_2$ is trifluoromethyl can be prepared from a compound of formula 39 wherein $R_2$ is iodo as follows: A compound of formula 39 wherein $R_2$ is iodo can be converted to a compound of formula

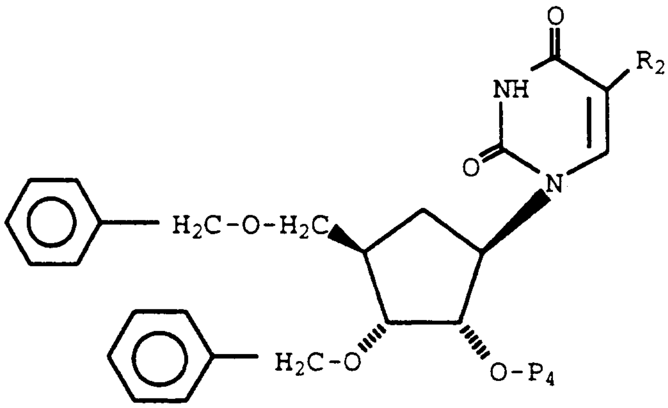

51

wherein $R_2$ is iodo and the protecting group $P_4$ is trityl, substituted trityl or acyl (e.g., acetyl). Treatment of this compound of formula 51 with trifluoromethyl iodide and copper according to procedures known in the art (see, for example, Y. Kobayashi, et al. J. Chem. Soc. Perkin, 2755 (1980); T. S. Lin et al., J. Med. Chem., 26, 1691(1983) and subsequent removal of the $P_4$ protecting group provides the compound of formula 39 wherein $R_2$ is trifluoromethyl. Oxidation of the compound of formula 39 wherein $R_2$ is trifluoromethyl and subsequent treatment with zinc/titanium tetrachloride/dibromomethane or methylenetriphenylphosphorane provides the compound of formula 41 wherein $R_2$ is trifluoromethyl. Removal of the benzyl protecting groups by treatment with boron trichloride provides the compound of formula 37 wherein $R_2$ is trifluoromethyl.

The compound of formula 44 wherein $R_2$ is trifluoromethyl can be prepared from the corresponding compound of formula 41 using methods known in the art (and analogous to those used in the conversion of compound 41 to compound 44 wherein, for example, $R_2$ is hydrogen, methyl, or ethyl).

Alternatively, the compound of formula 44 wherein $R_2$ is trifluoromethyl can be prepared from the corresponding compound of formula 37 by methods known in the art. See, for example, T. S. Lin et al., J. Med. Chem., 26, 1691(1983); P. Herdewijn, et al., J. Med. Chem., 28, 550 (1985); European Patent Applications EP 360018 and EP 204264.

The compound of formula 37 wherein $R_2$ is

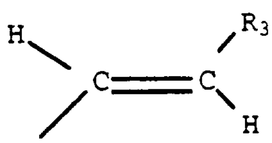

and $R_3$ is chloro, bromo, iodo, hydrogen, methyl or trifluoromethyl can be prepared from the compound of formula 39 wherein $R_2$ is iodo or -HgCl. Reaction of the compound of formula 39 wherein $R_2$ is iodo or -HgCl via organopalladium intermediates affords the compound of formula 39 wherein $R_2$ is

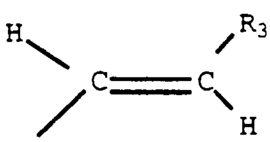

and $R_3$ is chloro, bromo, iodo, hydrogen, methyl or trifluoromethyl. The compound of formula 39 wherein $R_2$ is HgCl can be prepared from the compound of formula 39 wherein $R_2$ is hydrogen. See, for example, references in E. DeClercq et al., Pharmac. Ther., 26, 1 (1984); M.E. Perlman et al., J. Med. Chem., 28, 741 (1985); P. Herdewijn et al., J. Med., Chem., 28, 550 (1985); D. E. Bergstrom et al., J. Med. Chem. 27, 279 (1984).

Oxidation of a compound of formula 39 wherein $R_2$ is

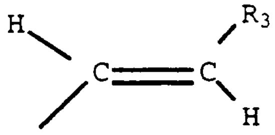

and $R_3$ is chloro, bromo, iodo, hydrogen, methyl, or trifluoromethyl, and subsequent treatment with zinc/titanium tetrachloride/dibromomethane or methylene-triphenylphosphorane provides the corresponding compound of formula 41. Removal of the benzyl protecting groups from this compound using boron trichloride affords the compound of formula 37 wherein $R_2$ is

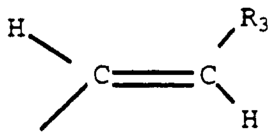

and $R_3$ is chloro, bromo, iodo, hydrogen, methyl or ttifluoromethyl.

The compound of formula 44 wherein $R_2$ is

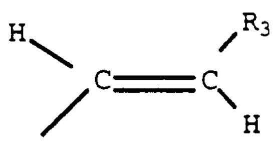

and $R_3$ is chloro, bromo, iodo, hydrogen, methyl or trifluoromethyl can be prepared from the corresponding compound of formula 37 by methods known in the art. See, for example, T. S. Lin et al., J. Med. Chem., 26, 1691(1983); P. Herdewijn, et al., J. Med. Chem., 28, 550 (1985); European Patent Applications EP 360018 and EP 204264.

Alternatively, the compound of formula 44 wherein $R_2$ is

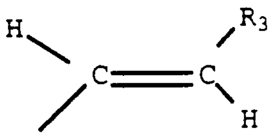

and $R_3$ is chloro, bromo, iodo, hydrogen, methyl or trifluoromethyl can be prepared from the corresponding compound of formula 41 using methods known in the art and analogous to those used in the conversion of compound 41 to compound 44 wherein, for example, $R_2$ is hydrogen, methyl or ethyl.

The compound of formula 37 wherein $R_2$ is ethynyl can be prepared from a compound of formula 51 wherein $R_2$ is iodo and protecting $P_4$ is acyl (e.g., acetyl), trityl, or substituted trityl. Treatment of the compound of formula 51 wherein $R_2$ is iodo and $P_4$ is acyl with trimethylsilylacetylene/bis (triphenylphosphine) palladium (II) chloride/copper (I) iodide in triethylamine and, subsequently, with sodium methoxide in methanol according to procedures known in the art [see, for example, E. DeClercq et al., J. Med. Chem., 26, 661 (1983)] provides the compound of formula 39 wherein $R_2$ is ethynyl. Alternatively, analogous treatment of the compound of formula 51 wherein $R_2$ is iodo and $P_4$ is trityl or substituted trityl, followed by removal of the trityl or substituted trityl protecting group $P_4$, using, for example, aqueous acetic acid or aqueous alcoholic mineral acid, provides the compound of formula 39 wherein $R_2$ is ethynyl. Oxidation of the compound of formula 39 followed by methylenation with zinc/titanium tetrachloride/dibromomethane or methylenetriphenylphosphorane yields the compound of formula 41 wherein $R_2$ is ethynyl, and subsequent removal of the benzyl protecting groups with boron trichloride provides the compound of formula 37 wherein $R_2$ is ethynyl.

The compound of formula 44 wherein $R_2$ is ethynyl can be prepared from the corresponding compound of formula 41 using methods known in the art and analogous to those used in the conversion of compound 41 to compound 44 wherein, for example, $R_2$ is hydrogen, methyl, or ethyl.

Alternatively, the compound of formula 44 wherein $R_2$ is ethynyl can be prepared from the corresponding compound of formula 37 by methods known in the art. See, for example T. S. Lin et al., J. Med. Chem., 26, 1691(1983); P. Herdewijn, et al., J. Med. Chem., 28, 550 (1985); European Patent Applications EP 360018 and EP 204264.

Compounds of formula 1 wherein $R_1$ is

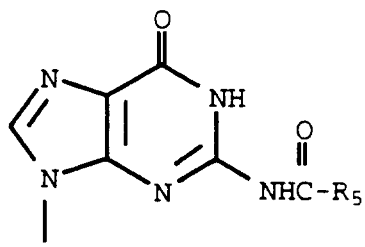

or

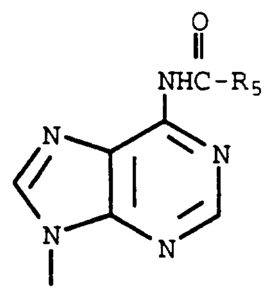

can be prepared from the corresponding compounds of formula 1 wherein $R_1$ is

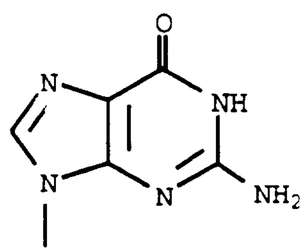

or

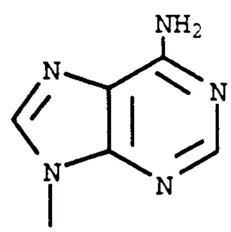

by methods known in the art.

Compounds of formula 1 wherein one or both $R_6$ and $R_7$ are

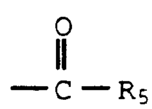

can be prepared by methods known in the art from the corresponding compounds of formula 1 wherein $R_6$ and $R_7$ are hydrogen.

For examples of acylation procedures see: "Synthetic Procedures in Nucleic Acid Chemistry", Vol.1, W.W. Zorbach and R.S. Tipson, Eds., John Wiley and Sons, 1968; "Nucleic Acid Chemistry," Part 1, L.B. Townsend and R. S. Tipson, Eds., John Wiley and Sons, 1978; Y, Ishido, et al., Nucleosides and Nucleotides, 5, 159 (1986); J.C. Martin, et al., J. Pharm. Sci., 76, 180 (1987); A Matsuda, et al., Synthesis, 385 (1986).

Compounds of formula 1 wherein $R_1$ is

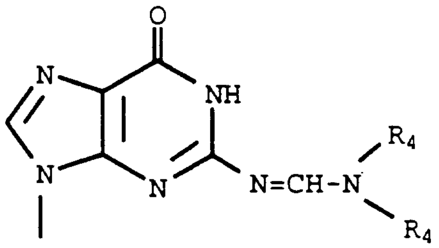

or

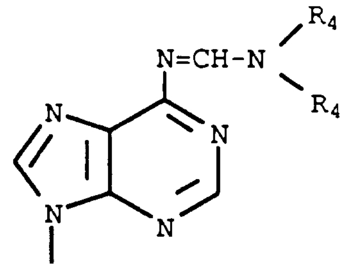

can be prepared from the corresponding compound of formula 1 wherein $R_1$ is

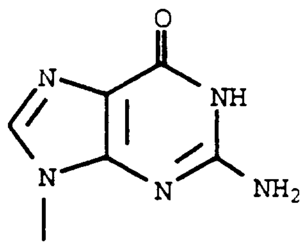

or

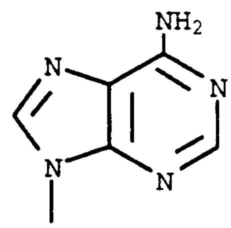

by procedures known in the art. See, for example, A. Holy and J. Zemlicka, Collect. Czech. Chem. Commun., 32, 3159 (1967); K,K, Ogilvie, et al., Nucleosides and Nucleotides, 4, 507 (1985); M. H. Caruthers, et al., J. Amer. Chem. Soc., 108, 2040 (1986).

Compounds of the formula 1 wherein $R_6$ and/or $R_7$ are $-PO_3H_2$ can be prepared from the corresponding compounds of formula 1 wherein $R_6$ and $R_7$ are hydrogen by procedures known in the art. See, for example, H. Schaller, et al., J. Amer. Chem. Soc., 85, 3821 (1963); J. Beres, et al., J. Med. Chem., 29, 494 (1986); R. Noyori, et al., Tet. Lett., 28, 2259 (1987); W. Pfeiderer, et al., Helv. Chim. Acta., 70, 1286 (1987); "Nucleic Acid Chemistry", Part 2, L. B. Townsend and R. S. Tipson, Eds., John Wiley and Sons, 1978.

Unless otherwise stated, the stereochemistry shown for the compounds of this invention and intermediates leading to compounds of this invention is relative and not absolute. It is drawn to show that in the compounds of this invention, the base represented by $R_1$ is cis to the $-CH_2OR_6$ substituent and trans to the $-OR_7$ substituent attached directly to the cyclopentyl ring.

The compounds of formula 1 wherein $R_1$ is

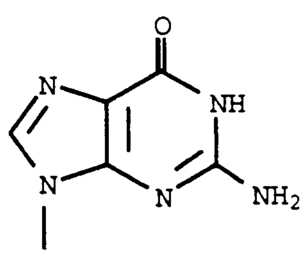

,

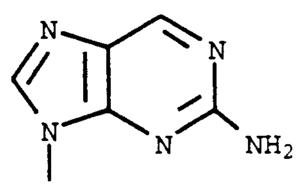

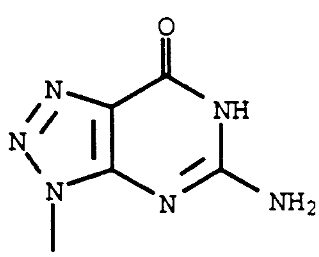

,

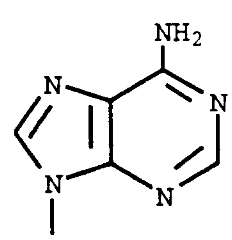

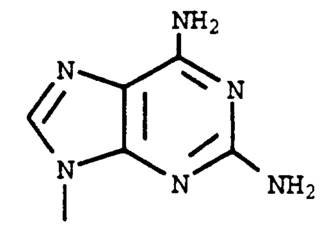

,

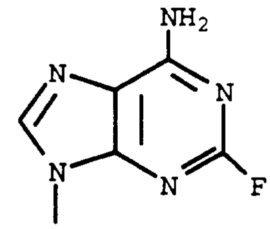

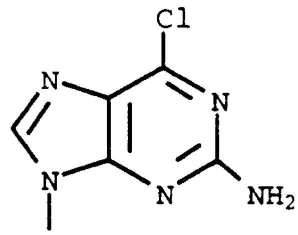

,

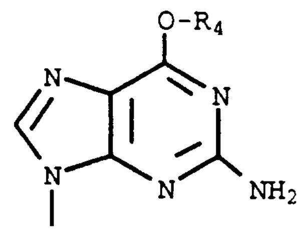

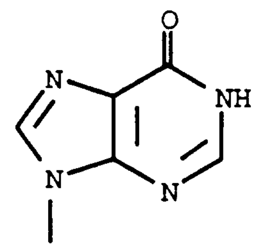

,

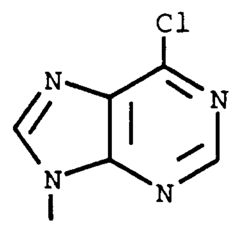

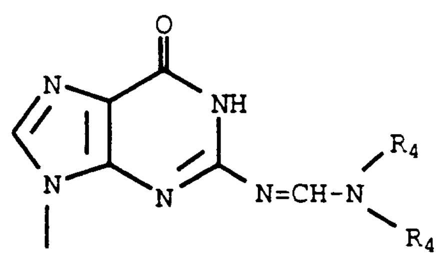

,

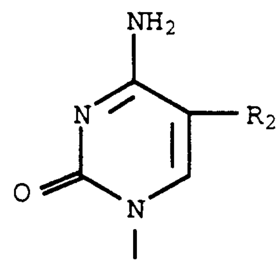

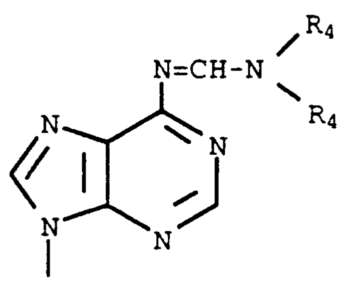

,

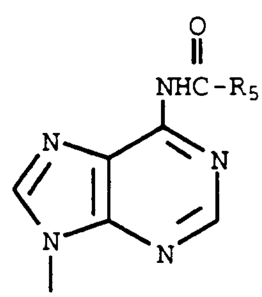

and

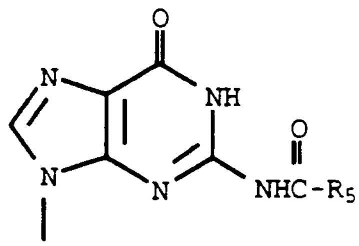

can form acid addition salts with inorganic or organic acids. Illustrative are the halide (e.g., chloride and bromide), alkylsulfonate, sulfate, phosphate and carboxylate salts.

The compounds of formula 1 wherein $R_1$ is

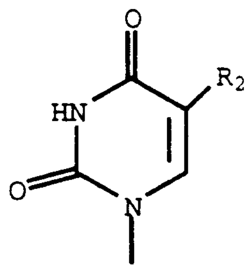

,

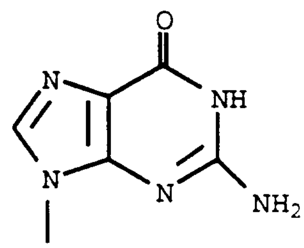

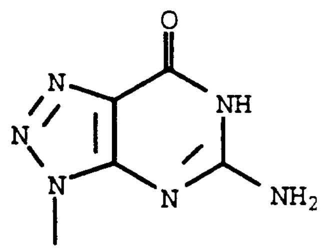

,

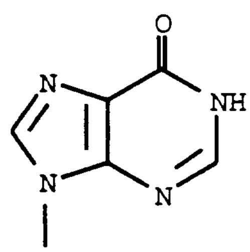

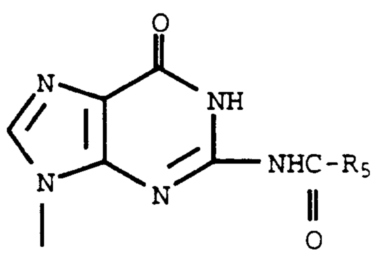

,

and

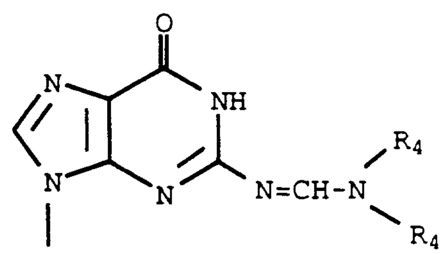

can form basic salts with inorganic and organic bases. Illustrative are alkali metal salts (e.g., sodium and potassium), alkaline earth metal salts (e.g., calcium and magnesium), ammonium and substituted ammonium salts.

The compounds of formula 1 wherein $R_6$ and/or $R_7$ are $-PO_3H_2$ can form basic salts with inorganic and organic bases. Illustrative are the alkali metal salts (e.g., sodium and potassium), alkaline earth metal salts (e.g., calcium and magnesium), ammonium and substituted ammonium salts.

The following examples are specific embodiments of this invention. All temperatures are given in degrees Centigrade.

Example 1

(1α,3β,4α)-2-Amino-1,9-dihydro-9-[3-hydroxy-4-(hydroxymethyl)-2-methylenecyclopentyl]-6H-purin-6-one

a) 3-[(Phenylmethoxy)methyl]cyclopentene

2-Cyclopentene-1-methanol [O. L. Chapman, et al., J. Amer. Chem. Soc., 100, 4878 (1978); A. Maercker and R. Geuss, Chem Ber., 106, 773 (1973)] (14.5 g., 0.147 mol.) was dried over 3°A molecular sieves, dissolved in dry dimethylformamide (80 ml) and transfered by canula into a stirred suspension of sodium hydride (7.06 g., 0.176 mol., 60% oil dispersion) in dry dimethylformamide (60 ml.) at 0°C. Benzyl bromide (10.9 ml., 0.09 mol.) was then added dropwise and the reaction mixture was allowed to warm to room temperature overnight. The reaction was quenched with cold water (150 ml), and the aqueous layer extracted three times with ethyl acetate (200 ml.). The combined organic layers were washed with brine, dried over sodium sulfate, filtered, and concentrated *in vacuo* to a yellow residue. This residue was purified by flash chromatography (Merck silica gel-60, 230-400 mesh, 4.5 1.), eluting with 1% acetone/hexane to give the title compound (19.1 g) as a colorless oil.

b). 2-[(Phenylmethoxy)methyl]-6-oxabicyclo[3.1.0]hexane

A solution of 3-[(phenylmethoxy)methyl]cyclopentene (0.495 g., 2.63 mmol) in paradioxane (3.37 ml) was added to a suspension of N-bromosuccinimide (0.562 g., 3.16 mmol.) in water (3.37 ml.) at 0°C. After 5 minutes, the reaction mixture was warmed to room temperature and stirred there for 3 hours. The reaction mixture was then concentrated *in vacuo* and partitioned between water and ether. The aqueous layer was extracted with ether and the combined organic layers were washed with water and brine, then dried over sodium sulfate. The volatiles were removed *in vacuo* to leave a yellow oil (0.389 g.). The residue was used in the subsequent reaction with no further purification.

The above residue (0.389 g.) was dissolved in methanol (4.3 ml.) under nitrogen, and potassium carbonate (0.473 g., 3.42 mmol.) was added. After 4 hours, the reaction mixture was concentrated *in vacuo* and the residue was partitioned between dichloromethane and water. The aqueous layer was extracted with dichloromethane and the combined organic layers were dried over magnesium sulfate and concentrated *in vacuo* to leave a yellow oil. The residue was purified by flash chromatography (Merck silica gel-60, 230-400 mesh, 50 ml.), eluting with 1% acetone/hexane to give the title compound (0.254 g.) as a colorless oil.

c) (trans)-5-[(Phenylmethoxy)methyl]-2-cyclopenten-1-ol

Sodium phenylselenide was prepared *in situ* by cautiously adding sodium borohydride to diphenyl diselenide (8.43 g., 0.027 mol.) in degassed absolute ethanol (150 ml.) until a colorless solution resulted. This solution (90 ml.) was added dropwise to a rapidly stirred solution of 2-[(phenylmethoxy)methyl]-6-oxabicyclo[3.1.0]hexane (5.0 g., 0.02 mol.) in degassed absolute ethanol (40 ml.) at ambient temperature. The reaction was refluxed at 90°C for 30 minutes, then cooled to room temperature and diluted with tetrahydrofuran (60 ml.). Following the dilution, the reaction was cooled further to 0°C and 30% hydrogen peroxide (25 ml.) was added dropwise. After completing the hydrogen peroxide addition, the reaction was allowed to warm to ambient temperature overnight, followed by heating at reflux for 2.5 hours. The reaction was cooled to room temperature and diluted with ethyl ether (250 ml.). The aqueous layer was removed and the organic layer was washed with saturated sodium bicarbonate and water. The organic layer was dried over sodium sulfate, filtered and concentrated *in vacuo* to give a yellow oil. The oil was purified by flash chromatography (Merck silica gel-60, 230-400 mesh, 1.5 l.), eluting with 30% ethyl acetate-hexane to give the title compound (2.35 g.) as an oil.

d) (1α,2β,3α,5α)-3-[(Phenylmethoxy)methyl]-6-oxabicyclo[3.1.0]hexan-2-ol

3-Chloroperoxybenzoic acid (4.90 g., 0.03 mol.) was added to a stirred suspension of the product from part (c) (2.35 g., 0.01 mol.) and sodium phosphate (8.09 g., 0.06 mol.) in dichloromethane (60 ml.) at 0°C. After being stirred at room temperature overnight, the reaction was diluted with saturated sodium bicarbonate and sodium thiosulfate. The organic solvent was removed and the aqueous layer was washed twice with ethyl acetate (75 ml.). The combined organic layers were washed with brine, dried over sodium sulfate and concentrated to a yellow oil. The oil was purified by flash chromatography (Merck silica gel-60, 230-400 mesh), eluting with 15% ethyl acetate-hexane, followed by 50% ethyl acetate-hexane, to give the title

compound (1.83 g.) as a colorless oil.

e) (1α,2β,3α,5α)-2-(Phenylmethoxy)-3-[(phenylmethoxy)methyl]-6-oxabicyclo[3.1.0]hexane

Sodium hydride (0.80 g, 19.61 mmol, 60% oil dispersion) was added to a solution of the product from part (d) (3.6 g., 16.34 mmol.) in dimethylformamide (17 ml.). The solution was then cooled to 0°C and benzyl bromide (2.33 ml., 19.61 mmol.) was added. After 3 days at ambient temperature, the reaction was diluted with water and the aqueous layer was extracted three times with ethyl acetate (200 ml). Drying the combined organic layers over sodium sulfate and concentrating *in vacuo* resulted in a yellow oil. The oil was purified by flash chromatography (Merck silica gel-60 230-400 mesh, 800 ml), eluting with 15% ethyl acetate-hexane to give the title compound (4.6 g.) as a yellow oil.

f) (1α,2α,3β,5β)-5-[2-Amino-6-(phenylmethoxy)-9H-purin-9-yl]-2-(phenylmethoxy)-3-[(phenylmethoxy)-methyl]-cyclopentanol

Lithium hydride (0.023 g., 2.9 mmol.) was added to a stirred solution of the product from part (e) (1.5 g., 4.83 mmol.) and 6-(phenylmethoxy)-9H-purin-2-amine (2.91 g., 12.08 mmol.) in dimethylformamide (23 ml.) at 70°C. The reaction mixture was then heated to 112°C for 3 days. After cooling, the reaction mixture was concentrated via Kugelrohr distillation under vacuum to yield a yellow oily-solid residue. The residue was purified by flash chromatography (Merck silica gel-60, 230-400 mesh), eluting with 1% methanol-dichloromethane to give the title compound (1.25 g.) as a yellow oil.

g) (1α,2α,3β,5β)-5-[2-[[(4-Methoxyphenyl)diphenylmethyl]amino]-6-(phenylmethoxy)-9H-purin-9-yl]-2-(phenylmethoxy)-3-(phenylmethoxy)methyl]cyclopentanol

Anisylchlorodiphenylmethane (1.23 g., 3.98 mmol.) was added to a 0°C solution of the product from part (f) (2.19 g., 3.98 mmol.) and triethylamine (0.55 ml., 3.98 mmol.) in dichloromethane (50 ml.) After allowing the reaction to warm to room temperature overnight, a second portion of anisylchlorodiphenylmethane (0.37 g., 0.11 mmol.) was added. After a further 2 hours, the reaction was quenched with saturated sodium bicarbonate, the organic layer removed, and the aqueous layer extracted twice with ethyl acetate (50 ml.). The combined organic layers were dried over sodium sulfate and concentrated *in vacuo* to a yellow oil. The oil was purified by flash chromatography (Merck silica gel-60, 230-400 mesh, 500 ml.), eluting with 50% ethyl acetate-hexane to afford the title compound (2.26 g.) as a colorless solid.

h) (2α,3β,5β)-5-[2-[[(4-Methoxyphenyl)diphenylmethyl]amino]-6-(phenylmethoxy)-9H-purin-9-yl]-2-phenylmethoxy-3-[(phenylmethoxy)methyl]cyclopentanone

To a dichloromethane (4 ml.) solution of oxalyl chloride (0.15 ml, 1.72 mmol.) at -78°C under argon was added dropwise dimethyl sulfoxide (0.25 ml., 3.53 mmol.). After being stirred at -78°C for 15 minutes, a dichloromethane (2 ml.) solution of the product from part (g) (0.484 g., 0.587 mmol.) was added dropwise. After a further 30 minutes, triethylamine (0.57 ml., 4.09 mmol.) was added and the reaction mixture was brought to 0°C for 3 minutes and then quenched with saturated sodium bicarbonate and brine. The aqueous layer was extracted three times with ethyl acetate. The combined organic layers were dried over sodium sulfate and evaporated *in vacuo* to leave the title compound (0.525 g.) as a slightly orange foam, which was azeotroped three times with dry toluene before use in the subsequent reaction.

i) Zinc - Titanium Tetrachloride - Dibromomethane Complex

This complex was prepared by the procedure of L. Lombardo, Tetrahedron Lett., 23, 4293 (1982). Titanium tetrachloride (3.85 ml., 0.035 mol.) was slowly added dropwise to a stirred mixture of zinc dust (9.8 g., 0.15 mol., activated by washing three times with 5% hydrochloric acid, then water, and finally ethyl ether) and dibromomethane (3.33 ml., 0.047 mol.) in dry tetrahydrofuran (100 ml.) at -45° to -49°C under argon. After the addition of the titanium tetrachloride was complete, the mixture was stirred at -45° to -49°C for 1 hour, at 0°C for 30 minutes, and then stirred at 5°C for four days. The slurry was stored at -20°C and warmed to room temperature prior to use.

j) (1α,3β,4α)-N-[(4-Methoxyphenyl)diphenylmethyl]-9-[2-methylene-3-(phenylmethoxy)-4-[(phenylmethoxy)-methyl)cyclopentyl]-9H-purin-2-amine

A slurry of zinc-titanium tetrachloride-dibromomethane complex from part (i) in tetrahydrofuran (4.3 ml., approximately 1.29 mmol.) was added to a stirred solution of the product from part (h) (0.395 g., less than 0.48 mmol.) in dichloromethane (3 ml.) under argon. The dark reaction mixture was stirred at room temperature for 2 hours, then a second portion of the zinc-titanium tetrachloride-dibromomethane complex (0.9 ml., approximately 0.27 mmol.) was added. After another hour, the mixture was added carefully to a rapidly stirred mixture of ethyl acetate and saturated sodium bicarbonate and stirred an additional 15 minutes. The aqueous layer was extracted three times with ethyl acetate, and the combined organic layers were dried over sodium sulfate. After evaporating the volatiles *in vacuo*, the crude residue was combined with the residue from a similar reaction starting with the product from part (h) (43.9 mg., 0.053 mmol.). The combined residues were purified by flash chromatography (Mallinkrodt SilicAR, 100-200 mesh silica gel, type 60A special, 3x 14 cm), eluting with ethyl acetate-hexanes (1:2, then 1:1) to give a semi-purified residue (218 mg.). This mixture was purified by flash chromatography (Merck silica gel-60, 230-400 mesh, 2cm x 18.5 cm), eluting with 7% ethyl acetate-dichloromethane to give the pure title compound (99 mg.) as an oily foam.

k) (1α,3β,4α)-2-Amino-1,9-dihydro-9-[3-hydroxy-4-(hydroxymethyl)-2-methylenecyclopentyl]-6H-purin-6-one.

To a dichloromethane (2.5 ml.) solution of the product from part (j) (282 mg., 0.344 mmol.) at -78°C under argon was added dropwise over several minutes a dichloromethane solution of boron trichloride (5.2 ml., 1 M in dichloromethane). The resulting orangish-brown mixture was stirred at -78°C for 1 hour, warmed to -10°C (ice-brine bath) for 5 minutes, and then a second portion of boron trichloride (1.5 ml., 1.5 mmol.) was added at -78°C. After 30 minutes at -78°C and 10 minutes at -10°C, the reaction mixture was removed from the ice-brine bath until a red homogeneous solution resulted. The reaction mixture was again cooled to -78°C, and a third portion of boron trichloride (1.5 ml., 1.5 mmol.) was added, the reaction kept at -78°C for 15 minutes, 10 minutes at -10°C, and recooled to -78°C before being cautiously quenched with methanol. After being stirred at room temperature for several minutes, the volatiles were concentrated *in vacuo* and the orange oil was co-evaporated three times with methanol to leave an orange glass. Water was added to the residue and the slurry was brought to pH 7. The resulting mixture was partially concentrated *in vacuo* and the aqueous suspension purified on a CHP-20P resin column (2x 10 cm), eluting first with water, then 3% acetonitrile-water to give the title compound (38.4 mg) as a colorless solid. Proton NMR (270 MHz, DMSO-$d_6$) δ : 10.51 (br s, 1 H), 7.60 (s, 1H), 6.35 (br s, 2H), 5.15 (d, J=6.4 Hz, 1H), 5.07-5.14 (m, 2H), 4.66 (s, 1H), 4.51 (t, J= 5.3 Hz, 1H), 4.21-4.26 (m, 1H), 3.58-3.65 (m, 1H), 3.37-3.45 (m, 1H), 2.14-2.21 (m, 1H), 1.64-1.85 (m, 2H); Carbon NMR (270 MHz, DMSO-$d_6$) δ: 156.8, 154.1, 153.3, 151.2, 135.6, 116.4, 109.6, 72.9, 61.6, 53.7, 47.2, 32.1; melting point = 146-158°C (dec.)

Example 2

(1α,2β,4α)-4-(6-Amino-9H-purin-9-yl)-2-hydroxy-3-methylenecyclopentanemethanol

a) (1α,2α,3β,5β)-5-(6-Amino-9H-purin-9-yl)-2-(phenylmethoxy)-3-[(phenylmethoxy)methyl]cyclopentanol

To a suspension of adenine (4.3 g., 31.8 mmol.) in dry dimethylformamide (70 ml.), at room temperature and under argon, was added in one portion sodium hydride dispersion (0.32 g., 8 mmol.,60% in oil). After being stirred for 1 hour, a solution of the product from Example 1(e) (3.3 g., 10.6 mmol.) in dry dimethylformamide (30 ml.) was added in one portion and the suspension was heated at 130°C for 48 hours. After cooling to room temperature the solvent was removed (30°C, high vacuum) to yield an oily solid residue. The residue was diluted with chloroform and the solids removed by filtration. The filtrate was evaporated to dryness and the residue co-evaporated with toluene several times. The semi-solid residue was diluted with dichloromethane and the insolubles again removed by filtration and the combined solids were extracted twice with warm ethyl acetate. The combined organics were concentrated *in vacuo* to yield a near white oily solid residue (6.4 g.). This material was purified by flash chromatography (Merck silica gel-60, 230-400 mesh, 2000 ml.), eluting with 5% methanol-chloroform to give the title compound (2.7 g.) as a white powder.

b) (1α,2β,3β,4α)-N-[9-[2-Hydroxy-3-(phenylmethoxy)-4-[(phenylmethoxy)methyl]cyclopentyl]-9H-purin-6-yl]-benzamide

To a solution of the product from part (a) (800 mg.) in dry pyridine (20 ml.), at room temperature and under argon, was added benzoyl chloride (0.836 ml., 7.2 mmol.). After 1 hour, the solvent was removed at room temperature *in vacuo* and the semi-solid residue taken up in ethyl acetate and washed with saturated sodium bicarbonate and twice with brine. Afer drying over magnesium sulfate, the removal of solvent yielded crude title compound as an orange oil.

This crude residue containing title compound was dissolved in ethanol (20 ml.), para-dioxane (20 ml.) and 1 N sodium hydroxide (10 ml.) and the solution was stirred at room temperature for 1 hour. The pH was then adjusted to 7.0 (from 13.5) with 1 N hydrogen chloride and the solution evaporated to dryness. The residue was taken up in ethyl acetate and the solution washed three times with brine, dried over magnesium sulfate, and the solvent removed to yield a yellow oil (1.41 g.). This material was purified by flash chromatography (Merck silica gel-60, 230-400 mesh, 150 ml.), eluting with 100% ethyl acetate to yield the title compound (759 mg.) as a white foam.

c) (1α,3β,4α)-N-[9-[2-Oxo-3-(phenylmethoxy)-4-[(phenylmethoxy)methyl]cyclopentyl]-9H-purin-6-yl]-benzamide

To a solution of 1,1,1-triacetoxy-1,1-dihydro-1,2-benziodoxol-3(1H)-one (1.75 g., 4.13 mmol.) in dry dichloromethane (20 ml.), at room temperature and under argon, was added in one portion neat tert-butanol (0.427 ml., 4.54 mmol.). After 15 minutes, a dichloromethane (5 ml.) solution of the product from part (b) (755 mg., 1.37 mmol.) was added. After a further 30 minutes, the reaction was poured into a mixture of sodium thiosulfate (4.5 g., 28 mmol.) in saturated sodium bicarbonate (20 ml.) and ethyl acetate (100 ml.) at 5°C. The mixture was vigorously stirred until a clear organic layer was obtained. The organic layer was separated and the aqueous layer washed with ethyl acetate. The combined organic layers were washed twice with brine, dried over magnesium sulfate, and the solvent removed *in vacuo* to yield the title compound (707 mg.) as a white foam. This material was unstable and used immediately without further purification.

d) (1α,3β,4α)-N-[9-[2-Methylene-3-(phenylmethoxy)-4-[(phenylmethoxy)methyl]cyclopentyl]-9H-purin-6-yl]-benzamide

To a solution of the product from part (c) (400 mg., 0.73 mmol.) in dichloromethane (10 ml.), at room temperature and under argon, was added in one portion a slurry of zinc-titanium tetrachloride-dibromomethane complex [prepared as described in Example 1(i)] in tetrahydrofuran (50 ml., approximately 15 mmol.). Stirring was continued for 30 minutes, after which the dark reaction was poured into a vigorously stirred mixture of saturated sodium bicarbonate (100 ml.) and ethyl acetate (100 ml.). Stirring was continued until the dark color was consumed (30 minutes). The clear colorless organic layer was separated and washed twice with brine, dried over magnesium sulfate, and the solvent removed *in vacuo* to yield an orange oil residue (378 mg.).

This material was combined with 362 mg of crude product from an identical reaction and the material purified by flash chromatography (Merck silica gel-60, 230-400 mesh, 500 ml.). Initial elution with 100% ethyl acetate afforded impure title compound (270 mg.) as a yellow foam. This material was purified by flash chromatography (Merck silica gel-60, 230-400 mesh, 500 ml.), eluting with 60% ethyl acetate-hexane, followed by 70% ethyl acetate hexane to give the pure title compound (105 mg.) as a white foam.

e) (1α,3β,4α)-[9-[2-Methylene-3-(phenylmethoxy)-4-{(phenylmethoxy)methyl]cyclopentyl]-9H-purin-6-amine

To a solution of the product from part (d) (105 mg., 0.19 mmol.) in ethanol (1 ml.) was added 1N sodium hydroxide (0.5 ml.) and the resulting orange solution was stirred at room temperature overnight. Additional 1 N sodium hydroxide (1 ml.) was added, followed by sufficient para-dioxane to dissolve the oil obtained on addition of the sodium hydroxide. Stirring was continued for 1.5 days. The pH was adjusted to 7.0 (from 12.5) with 1 N hydrogen chloride. The organic solvents were removed and the cloudy aqueous residue extracted three times with ethyl acetate. The combined extracts were washed twice with brine, dried over magnesium sulfate, and the solvent removed to yield a viscous oil (80 mg.). This material was combined with 140 mg. of impure product previously obtained and purified by flash chromatography (Merck silica gel-60, 230-400 mesh, 280 ml), eluting with 5% methanol-ethyl acetate to yield the pure title

compound (123 mg.) as a white foam.

f) (1α,2β,4α)-4-(6-Amino-9H-purin-9-yl)-2-hydroxy-3-methylenecyclonentanemethanol

To a solution of the product from part (e) (150 mg., 0.34 mmol.) in dry dichloromethane (3 ml.), at -78° C and under argon, was added dropwise boron trichloride (1 ml., 1 M in dichloromethane). Stirring was continued at -78° C for 1 hour, after which the reaction was briefly warmed to 0° C, then re-cooled to -78° C. The reaction was quenched by the dropwise addition of methanol (0.5 ml.) and then allowed to warm to room temperature. The solvent was removed and the residue diluted with water. The pH was adjusted to 7.0 with 1 N sodium hydroxide. The cloudy suspension was chromatographed on a CHP-20P resin column (95 ml.) . After initial elution with water, continued elution with 5% acetonitrile-water afforded a clear, pale yellow glass (51 mg.). This material was futher purified by semi-preperative HPLC on a Waters Assoc C-18 Prep-Pak Cartridge (25x10 cm). Elution with 5% acetonitrile-water gave a glass, which was triturated with methanol to give a white powder (35 mg.). This material was recrystallized from methanol to yield the pure title compound (20 mg.) as a white powder. Proton NMR (270 MHz, DMSO-$d_6$): δ : 8.15 (s, 1H), 8.12 (s, 1H), 7.21 ( s, 2H ), 5.39 (m, 1H ), 5.26 (d, 1H), 5.18 (s, 1H), 4.71 (s, 1H), 4.60 (t, 1H ), 4.38 (m, 1H), 3.70 (m, 1H), 3.50 (m, 1H), 2.30 (m, 1H), 1.98 (m, 1H ), 1.90 (m, 1H); m.p. 218-219° C (dried at 90° C for 24 hours under high vacuum).

| Anal. calc'd. for $C_{12}H_{15}N_5O_2$ • 0.12 $CH_3OH$: | | | |
|---|---|---|---|
| | C, 54.96; | H, 5.89; | N, 26.40 |
| Found: | C, 55.01; | H, 5.48; | N, 26.00. |

Example 3

(1α,3β,4α)-1-[3-Hydroxy-4-(hydroxymethyl)-2-methylenecyclopentyl]-5-methyl-2,4(1H,3H)-pyrimidinedione

a) (1α,2β,3β,4α)-1-[2-Hydroxy-3-(phenylmethoxy)-4-[(phenylmethoxy)methyl]cyclopentyl]-5-methyl-2,4-(1H,3H)-pyrimidinedione

To a suspension of thymine (3.4 g., 27 mmol.) in dry dimethylformamide (100 ml.), at room temperature and under argon, was added sodium hydride dispersion (0.29 g., 7.2 mmol., 60% in oil) in one portion. The mixture was heated at 60° C for 30 minutes to yield a milky reaction mixture to which was added a dimethylformamide (50 ml.) solution of the product from Example 1(e) (2.8 g., 9 mmol.). Heating was continued at 140° C for 4 days. After cooling to room temperature, the solvent was removed (30° C, high vacuum), the oily solid residue diluted with chloroform, and the insolubles removed by filtration. The filtrate was evaporated to dryness and the dark oil residue co-evaporated twice with toluene. The resulting dark oil was purified by flash chromatography (Merck silica gel-60, 230-400 mesh, 2.2 l.), eluting with 80% ethyl acetate-hexane, followed by 100% ethyl acetate, to afford the title compound (1.5 g.) as a white foam.

b) (1α,3β,4α)-5-Methyl-1-[2-oxo-3-(phenylmethoxy)-4-[(phenylmethoxy)methyl]cyclopentyl]-2,4(1H,3H)-pyrimidinedione

To a solution of 1,1,1-triacetoxy-1,1-dihydro-1,2-benziodoxol-3(1H)-one (1.46 g., 3.45 mmol.) in dry dichloromethane (20 ml.), at ambient temperature and under argon, was added in one portion tert-butanol (0.358 ml., 3.8 mmol.), followed by a solution of the product from part (a) (500 mg., 1.15 mmol.) in dichloromethane (10 ml.). The resulting solution was stirred for an additional 45 minutes. The reaction mixure was poured into a well stirred mixture of 10% sodium thiosulfate (15 ml.), saturated sodium bicarbonate (15 ml.), brine (15 ml.), and ethyl acetate (150 ml.). Stirring was continued until a clear solution was obtained and the aqueous layer was separated and washed with ethyl acetate. The combined organic layers were washed twice with brine, dried over magnesium sulfate, and the solvent removed *in vacuo* to yield the title compound (519 mg.) as a white solid. This material was used immediately without further purification.

c) (1α,3β,4α)-5-Methyl-1-[2-methylene-3-(phenylmethoxy)-4-[(phenylmethoxy)methyl]cyclopentyl]-2,4-(1H,3H)-pyrimidinedione

To a solution of the product from part (b) (500 mg., less than 1.15 mmol.) in dry dichloromethane (15 ml.), at room temperature and under argon, was added in one portion a slurry of zinc-titanium tetrachloride-dibromomethane complex in tetrahydrofuran, prepared as described in Example 1(i), (15 ml., approximately 4.5 mmol). The dark reaction mixture was stirred for 45 minutes, then poured into a vigorously stirred mixture of saturated sodium bicarbonate (50 ml.) and ethyl acetate (100 ml.). Vigorous stirring was continued until the dark color was consumed (approximately 45 minutes). The clear colorless organic layer was separated and the aqueous layer was washed twice with ethyl acetate (50 ml.). The combined organic layers were washed twice with brine, dried over magnesium sulfate, and the solvent removed *in vacuo* to yield a viscous foam residue (470 mg.). This material was purified by flash chromatography (Merck silica gel-60, 230-400 mesh, 200 ml.), eluting with 50% ethyl acetate-hexane to give the title compound (158 mg.) as a white foam.

d) (1α,3β,4α)-1-[3-Hydroxy-4-(hydroxymethyl)-2-methylenecyclopentyl]-5-methyl-2,4(1H,3H)-pyrimidinedione

To a solution of the product from part (c) (280 mg., 0.65 mmol.) in dry dichloromethane (5 ml.), at -78°C and under argon, was added dropwise boron trichloride (1.95 ml, 1 M in dichloromethane). After 15 minutes at -78°C, the reaction was quenched by the dropwise addition of methanol (0.5 ml.) and the resulting clear colorless solution was allowed to warm to room temperature. The solvent was removed and the residue was co-distilled twice with methanol. The residue was taken up in water and the pH adjusted to 7.0 (from approximately 2) with 1 N sodium hydroxide. The aqueous suspension was chromatographed on a column of CHP-20P resin (70 ml.). After initial elution with water, continued elution with 3% acetonitrile-water yielded crude (1α,3β,4α)-1-[3-hydroxy-4-(hydroxymethyl)-2-methylene-cyclopentyl]-5-methyl-2,4-(1H,3H)-pyrimidinedione as a white solid (121 mg.). This material was combined with 74 mg of crude (1α,3β,4α)-1-[3-hydroxy-4-(hydroxymethyl)-2-methylene-cyclopentyl]-5-methyl-2,4(1H,3H)-pyrimidinedione, obtained from a similar reaction [starting with the product from part (c), (160 mg., 0.37 mmol.)], and recrystallized from warm methanol-acetonitrile to give the title compound (155 mg.) as a white powder. Proton NMR (270 MHz, DMSO-$d_6$): δ: 11.27, (br s, 1H), 7.30 ( d, J=1.2 Hz, 1H), 5.28 (m, 1H), 5.23 ( d, J=6.5 Hz, 1H), 5.18 (d, 1H), 4.87 (d, 1H), 4.55 (m, 1H), 4.19 (m, 1H), 3.66 (m, 1H), 3.44 (m, 1H), 2.06 (m, 1H), 1.76 (m, 1H), 1.49 (q, J=11.7 Hz 1H); melting point = 212-213°C (dried 24 hours at 90°C under high vacuum).

An analytical sample of the title compound was obtained by recrystallization from warn acetonitrile to yield white crystals, m.p., 213-214°C, (dried 24 hours at 90°C under high vacuum).

| Anal. calc'd. for $C_{12}H_{16}N_2O_4$: | | | |
|---|---|---|---|
| | C, 57.13; | H, 6.39; | N, 11.10 |
| Found: | C, 56.91; | H, 6.40; | N, 11.10. |

Example 4

(1α,3β,4α)-1-[3-Hydroxy-4-(hydroxymethyl)-2-methylenecyclopentyl]-5-iodo-2,4(1H,3H)-pyrimidinedione

a). Uracil, mono-tetrabutylammonium salt

Aqueous tetrabutylammonium hydroxide (2.7 ml., 42% solution, 4.32 mmol.) was added to uracil (1 g., 8.93 mmol.) in dry dimethylformamide (14 ml.) at room temperature. The reaction mixture was stirred for 1 hour, the volatiles removed *in vacuo* , and the residue co-evaporated four times with dry dimethylformamide to give the title compound (1.97 g.) as a white powder.

b) (1α,2β,3β,4α)-1-[2-Hydroxy-3-(phenylmethoxy)-4-[(phenylmethoxy)methyl]cyclopentyl]-2,4(1H,3H)-pyrimidinedione

The product from Example 1 (e) (3.0 g., 9.7 mmol.) and the mono-tetrabutylammonium salt of uracil (18 g., 0.038 mol.) were combined in dimethylformamide (60 ml.) and heated to 110°C for 48 hours. The

mixture was then cooled to room temperature, diluted with water (100 ml.), and the aqueous layer was extracted three times with ethyl acetate (100 ml.). The combined organic layers were dried over sodium sulfate, filtered, and concentrated *in vacuo* to an off white solid. The solid residue was dissolved in ethyl acetate-methanol and pre-adsorbed onto Baker's silica gel (60-200 mesh), which was then loaded onto a silica gel column (Merck silica gel-60, 230-400 mesh, 1 l.). Elution with 100% ethyl acetate gave the title compound (2.5 g.) as an oil .

Alternatively, the title compound was also prepared by adding lithium hydride (1 mg., 0.125 mmol.) to a mixture of the product from Example 1(e) (130 mg., 0.419 mmol.) and uracil (141 mg., 1.26 mmol.) in dry dimethylformamide (1.95 ml.) at room temperature under argon. The mixture was heated at 140°C for 24 hours and then concentrated *in vacuo* to a dark brown solid. This material was purified by flash chromatography (Merck silica gel-60, 230-400 mesh, 100 ml.), eluting with ethyl acetate to give the title compound (78 mg.) as an oil.

c) (1α,2β,3β,4α)-1-[2-Hydroxy-3-(phenylmethoxy)-4-[(phenylmethoxy)methyl]cyclopentyl]-5-iodo-2,4(1H,3H)-pyrimidinedione

Nitric acid (0.5 N, 9.48 ml.) and iodine (1.19 g., 4.7 mmol.) were added to a solution of the product from part (b) (1.0 g., 2.4 mmol.) in para-dioxane (24 ml.) at room temperature. After heating the mixture for 2 hours at 90°C, the reaction was cooled to room temperature and diluted with water. The reaction mixture was neutralized with sodium bicarbonate and extracted four times with dichloromethane (15 ml.). The combined organic layers were concentrated to a red residue, which was co-evaporated four times with a mixture of chloroform-water-methanol to yield a light yellow solid. The solid residue was purified by flash chromatography (Merck silica gel-60, 230-400 mesh), eluting with 50% ethyl acetate-hexane to yield the title compound (1.1 g.) as a yellow foam.

d) (1α,3β,4α)-5-Iodo-1-[2-oxo-3-(phenylmethoxy)-4-[(phenylmethoxy)methyl]cyclopentyl]-2,4(1H,3H)-pyrimidinedione

tert-Butanol (0.05 ml., 0.5 mmol.) was added to a rapidly stirring suspension of 1,1,1-triacetoxy-1,1-dihydro-1,2-benziodoxol-3(1H)-one (0.695 g., 1.6 mmol.) in dichloromethane (3.5 ml.) at room temperature under argon. The mixture remained at room temperature for 15 minutes, then a dichloromethane (4 ml.) solution of the product from part (c) (0.50 g., 0.91 mmol.) was added. After 30 minutes, the reaction was added to a stirring mixture of brine (5 ml.), 10% aqueous sodium thiosulfate (5 ml.), and saturated sodium bicarbonate (5 ml.). After stirring at room temperature for 30 minutes, the mixture was diluted with ethyl acetate and the aqueous layer was extracted twice with ethyl acetate. The combined organic layers were concentrated *in vacuo* to yield the impure title compound (0.498 g.) as a white solid.

e) (1α,3β,4α)-5-Iodo-1-[2-methylene-3-(phenylmethoxy)-4-[(phenylmethoxy)methyl]cyclopentyl]-2,4(1H,3H)-pyrimidinedione

The product from part (d) was azetropically dried by short path distillation of dry toluene and the resulting residue was dissolved in dichloromethane (3 ml.). A slurry of zinc-titanium tetrachloride-dibromomethane complex in tetrahydrofuran prepared as described in Example 1 (i) (8.4 ml., approximately 2.5 mmol.) was added to the dichloromethane solution at room temperature. After 6 hours, the reaction was added to a stirred mixture of ethyl acetate and saturated sodium bicarbonate. After 1 hour, the aqueous layer was extracted twice with ethyl acetate (50 ml.) and the combined organic layers were concentrated *in vacuo* to give a yellow residue. The residue was purified by flash chromatography (Merck silica gel-60, 230-400 mesh), eluting with 2% methanol-dichloromethane to give the title compound (0.165 g.) as an off white solid.

f) (1α,3β,4α)-1-[3-Hydroxy-4-(hydroxymethyl)-2-methylenecyclopentyl]-5-iodo-2,4(1H,3H)-pyrimidinedione

To a stirred dichloromethane (6 ml) solution of the product from part (e) (0.165 g., 0.30 mmol.) at -78°C was added dropwise a dichloromethane solution of boron trichloride (3 ml., 3.0 mmol.). After 10 minutes, the reaction mixture was removed from the carbon dioxide/acetone bath for 15 minutes, resulting in a homogenous solution. The resulting solution was re-cooled to -78°C and stirred an additional 2 hours. The reaction was quenched at 0°C by the dropwise addition of methanol, warmed to room temperature, and concentrated *in vacuo*. The resulting residue was co-evaporated three times from methanol to give a red

oil. The oil was taken up in methanol, diluted with water and neutralized with 0.1 N potassium hydroxide. The volatiles were then removed *in vacuo* , and the resulting residue was slurried in water and purified by CHP-20P resin chromatography, eluting with water, followed by 2% methanol-water, to give the title compound (58 mg.) as a coloroless solid. Proton NMR (270 MHz, DMSO-$d_6$): δ: 11.55 (br s, 1H), 7.84 (s,1H), 5.09-5.18 (m, 3H), 4.86 (s, 1H), 4.43-4.45 (m, 1H), 4.13-4.16 (m, 1H), 3.4-3.6 (m, 2H), 1.9-2.05 (m, 1H), 1.65-1.73 (m, 1H), 1.48-1.53 (m, 1H); melting point = dec. 232 °C.

Example 5

[1α(E),3β,4α]-5-[2-Bromoethenyl)-1-[3-hydroxy-4-(hydroxymethyl)-2-methylenecyclopentyl]-2,4-(1H,3H)-pyrimidinedione

a) [1α(E),2β,3β,4α]-3-[1,2,3,4-Tetrahydro-1-[2-hydroxy-3-(phenylmethoxy)-4-[(phenylmethoxy)methyl]-cyclopentyl]-2,4-dioxo-5-pyrimidinyl]-2-propenoic acid, methyl ester

Triethylamine (1.34 ml., 9.61 mmol.), triphenylphosphine (0.15 g., 0.57 mmol.) and palladium(II) acetate (0.05 g.) were combined in dry dimethylformamide (17 ml., deoxygenated), then heated to 70 °C resulting in a red solution. After 15 minutes, a solution of (1α,2β,3β,4α)-1-[2-hydroxy-3-(phenylmethoxy)-4-[-(phenylmethoxy)methyl]cyclopentyl]-5-iodo-2,4(1H,3H)-pyrimidinedione, prepared as described in Example 4 (c) ( 1.5 g., 2.74 mmol.) in dimethylformamide (10 ml., deoxygenated) was added, followed by methyl acrylate (0.80 ml., 9.65 mmol.). After 8 hours, the reaction was cooled to room temperature and concentrated *in vacuo* to a yellow oil. This residue was combined with crude product from a similar reaction starting with the product of Example 4 (c) ( 2.1 g., 2.39 mmol.). The combined residues were purified by flash chromatography (Merck silica gel-60, 230-400 mesh), eluting with 2% methanol-dichloromethane to give the title compound (2.1 g.) as a yellow foam.

b) [1α(E),2β,3β,4α]-3-[1,2,3,4-Tetrahydro-1-[2-hydroxy-3-(phenylmethoxy)-4-[(phenylmethoxy)methyl]-cyclopentyl]-2,4-dioxo-5-pyrimidinyl]-2-propenoic acid

2 N Potassium hydroxide (16.3 ml.) was added at room temperature to a tetrahydrofuran (23 ml.) solution of the product from part (a) (1.62 g., 3.26 mmol.). The reaction was stirred at room temperature for 6 hours and then a second portion of 2 N potassium hydroxide (4 ml.) was added. After 1 hour, the reaction mixture was cooled to 0 °C and brought to pH 2 with 3 N hydrogen chloride. The solution was then diluted with ethyl acetate and the aqueous layer extracted three times with ethyl acetate. The combined organic layers were dried over sodium sulfate and concentrated *in vacuo* to give the title compound (1.6 g.) as an orange solid. This material was used without further purification.

c) [1α(E),2β,3β,4α]-5-(2-Bromoethenyl)-1-[2-hydroxy-3-(phenylmethoxy)-4-[(phenylmethoxy)methyl]-cyclopentyl]-2,4(1H,3H)-pyrimidinedione

A 0.5 M solution of N-bromosuccinimide in dimethylformamide (6.1 ml., 3.05 mmol.) was added to a mixture of the product from part (b) (1.6 g., 3.26 mmol., dried by azeotropic distillation with dimethylformamide) and potassium bicarbonate (1.05 g., 10.43 mmol.) in dimethylformamide (18 ml.) at room temperature . After 2 hours, the reaction mixture was concentrated *in vacu*o, taken up in dichloromethane, and washed with dilute sodium thiosulfate, 0.1 N potassium bicarbonate, and then water. The organic layer was dried over sodium sulfate and concentrated *in vacuo* to a yellow oil . The oil was purified by flash chromatography (Merck silica gel-60, 230-400 mesh), eluting with 1% ethanol-dichloromethane, followed by 2% ethanol-dichloromethane, to afford the title compound (0.75 g.) as an off white solid.

d) [1α(E),3β,4α]-5-(2-Bromoethenyl)-1-[2-oxo-3-(phenylmethoxy)-4-[(phenylmethoxy)methyl]cyclopentyl]-2,4-(1H,3H)-pyrimidinedione

tert-Butanol (0.043 ml.) was added to a stirred suspension of 1,1,1-triacetoxy-1,1-dihydro-1,2-benziodoxol-3(1H)-one (0.59 g., 1.38 mmol.) in dichloromethane (3 ml.) at room temperature. After 15 minutes, a solution of the product from part (c) (0.40 g., 0.77 mmol.) in dichloromethane (3 ml.) was added. After another 2 hours, the reaction was added to a vigorously stirred mixture of brine (10 ml.), 10% aqueous sodium thiosulfate (10 ml.), and saturated sodium bicarbonate (10 ml.). After 1 hour, the reaction was diluted with ethyl acetate, and the aqueous layer was extracted twice with ethyl acetate. The combined organic

layers were concentrated *in vacuo* to give crude title product as a white solid. This residue was combined with crude title product from a similar reaction starting with the product from part (c) (0.348 g., 0.66 mmol.). This combined residue was re-dissolved in dichloromethane and added to a vigorously stirred mixture of brine (20 ml.), 10% aqueous sodium thiosulfate (20 ml.), and saturated sodium bicarbonate (20 ml.). After 1 hour, the reaction was diluted with ethyl acetate and the aqueous layer was extracted three times with ethyl acetate. The combined organic layers were concentrated *in vacuo* to yield 0.75 g. of the crude title compound as a yellow solid. This material was used without further purification.

e) [1α(E),3β,4α]-5-(2-Bromoethenyl)-1-[2-methylene-3-(phenylmethoxy)-4-[(phenylmethoxy)methyl]-cyclopentyl]-2,4(1H,3H)-pyrimidinedione

A slurry of zinc-titanium tetrachloride-dibromomethane complex, prepared as described in Example 1 (i), in tetrahydrofuran (18 ml., approximately 5.5 mmol.) was added to a solution of the product from part (d) (approximately 0.75 g., approximately 1.43 mmol.) in dichloromethane (14 ml.) at room temperature under argon. After 45 minutes, a second portion of the zinc-titanium tetrachloride-dibromomethane complex (9 ml., approximately 2.79 mmol.) was added to the reaction and the mixture stirred at room temperature for an additional 1 hour before storing at -40°C overnight. After the reaction mixture had warmed to ambient temperature, a third portion of the zinc-titanium tetrachloride-dibromomethane complex (9 ml., approximately 2.79 mmol.; total 36 ml., approximately 11.1 mmol.) was added. After an additional hour, the reaction mixture was added to a rapidly stirred solution of ethyl acetate and saturated sodium bicarbonate. The mixture was stirred at room temperature for 1 hour, then diluted with ethyl acetate. The aqueous layer was extracted three times with ethyl acetate and the combined organic layers were dried over sodium sulfate, filtered and concentrated *in vacuo* to a colorless oil. The resulting residue was purified by flash chromatography (Merck silica gel-60, 230-400 mesh), eluting with 10% ethyl acetate-dichloromethane to afford a colorless foam (0.168 g.). This material was further purified by flash chromatography (Merck silica gel-60, 230-400 mesh), eluting with 10% ethyl acetate-dichloromethane to give the pure title compound (0.1 g.) as a colorless foam.

f) [1α(E),3β,4α]-5-(2-Bromoethenyl)-1-[3-hydroxy-4-(hydroxymethyl)-2-methylenecyclopentyl]-2,4(1H,3H)-pyrimidinedione

Boron trichloride (1.9 ml., 1 M solution in dichloromethane) was added dropwise to a stirred solution of the product from part (e) (0.10 g., 0.19 mmol.) in dichloromethane (3.5 ml.) at -78°C under argon. After 10 minutes, the solution was warmed to ambient temperature until a homogeneous solution resulted (approximately 15 minutes), then re-cooled to -78°C. After an additional 1.5 hours, the reaction mixture was quenched at O°C by the dropwise addition of methanol. The volatiles were removed *in vacuo* and the orange oily residue was co-evaporated three times with methanol. The resulting residue was taken up in water and neutralized with potassium hydroxide before concentrating to a slurry. The slurry was loaded onto a CHP-20P resin column and initially eluted with water, followed by a gradient of acetonitrile-water (1, 3, 5, 7, 9, 15, 20, 30, and then 40% acetonitrile) to give the title compound (0.024 g) as a colorless solid. Proton NMR (270 MHz, DMSO-$d_6$): δ: 11.50 (br s, 1H), 7.20 (d J= 13.6 Hz, 1H), 6.83 (d, J= 13.6 Hz,1H), 5.14-5.23 (m, 2H), 5.12 (d, J= 6.8 Hz,1H), 4.86 (s, 1H), 4.4-4.5 (m, 1H), 4.13-4.16 (m, 1H), 3.6-3.65 (m, 1H), 3.3-3.4 (m, 1H), 1.95-2.05 (m, 1H), 1.65-1.75 (m, 1H), 1.40-1.50 (m, 1H); melting point = dec. 190°C.

Example 6

Treatment of Viral Infection In Cell Culture in Vitro

Assays were performed in cell culture systems to determine the concentrations of compounds that are effective in preventing several kinds of viral infections. The assays are described below, and the results presented in Table 1.

Abbreviations:

HSV-1 (herpes simplex virus type 1, strain Schooler), HSV-2 (herpes simplex virus type 2, strain 186), VZV (varicella zoster virus, strain ELLEN), HCMV (human cytomegalovirus, strain AD 169).

Cell Culture Assays:

HSV-1, HSV-2, HCMV and VZV antiviral assays: Virus was adsorbed to WI-38 cell culture monolayers in 6 well culture plates (Costar, Cambridge, MA) for 1 hour prior to addition of maintenance medium containing duplicate dilutions of the test compound. Inhibition of plaque development was evaluated on fixed and stained monolayers after 4 days incubation at 37°C. for HSV-1 and HSV-2, and after 5 - 7 days incubation at 37°C. for HCMV and VZV. $ID_{50}$ values were determined from the drug concentration which conferred at least a 50% plaque reduction compared to virus controls.

Table 1

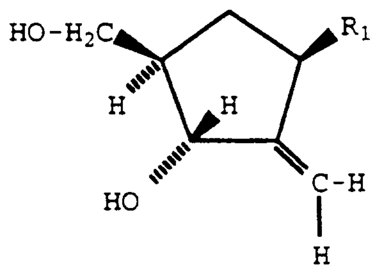

| | $ID_{50}$ (μM) for the following viruses | | | |
|---|---|---|---|---|
| $R_1$ | HSV-1 | HSV-2 | VZV | HCMV |
| 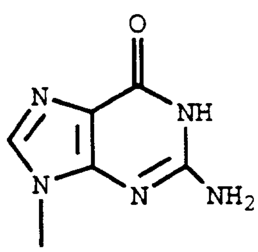 | 4-7[1] | 7-18[1] | 7-18[1] | 7[1] |
| 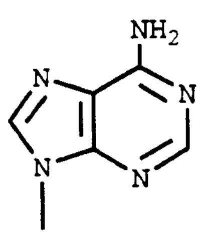 | 383[2] | 383[2] | >38[2] | >38[2] |
| 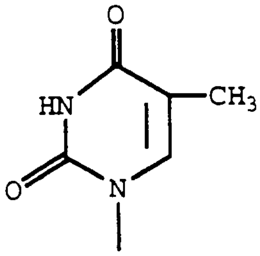 | 100-200[3] | >396[3] | 40[3] | >396[3] |

| | | | | |
|---|---|---|---|---|
| 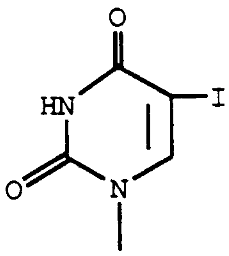 | >275 | >275 | 275 | 28-275[4] |
| 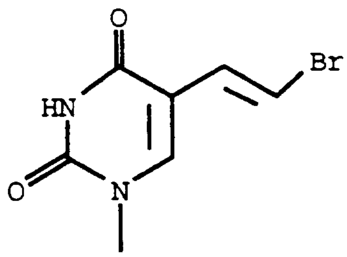 | 291[5] | >291[5] | 1.5-3 | ND[6] |

1 Visible toxicity to the cell monolayers under the assay conditions at 361μM.

2 Visible toxicity to the cell monolayers under the assay conditions at 383μM.

3 Visible toxicity to the cell monolayers under the assay conditions at 396μM.

4 Visible toxicity to the cell monolayers under the assay conditions at 275μM.

5 Visible toxicity to the cell monolayers under the assay conditions at 291μM.

6 Not determined.

## Claims

**1.** A compound having the formula

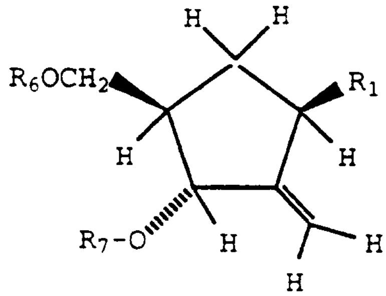

or a pharmaceutically acceptable salt thereof wherein $R_1$ is

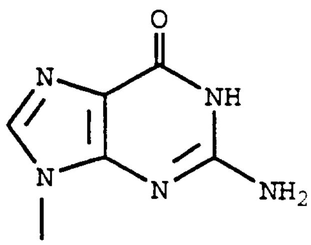 ,

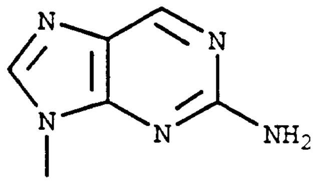 ,

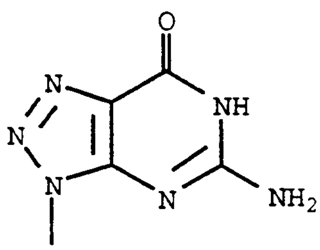 ,

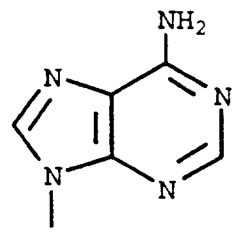 ,

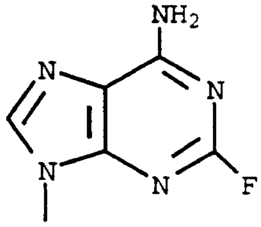 ,

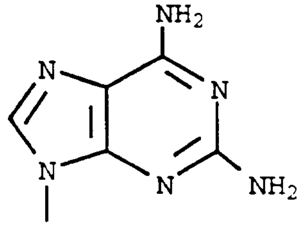 ,

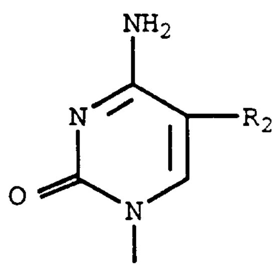 , 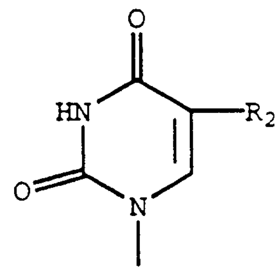 ,

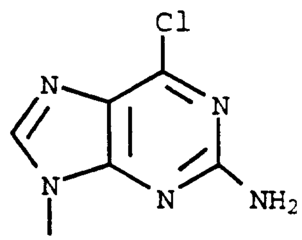 , 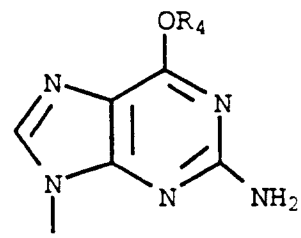 ,

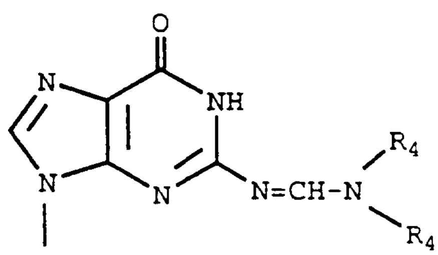 , 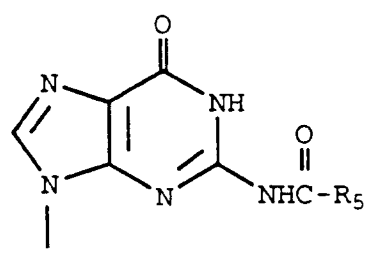 ,

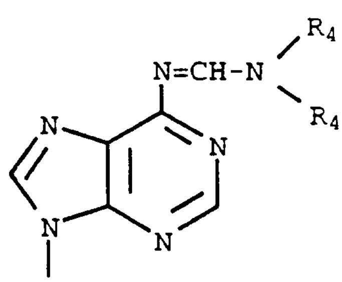 , 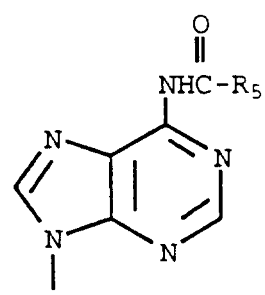 ,

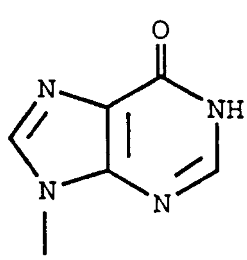 , 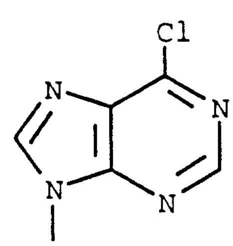 .

wherein $R_2$ is fluoro, chloro, bromo, iodo, hydrogen, methyl, trifluoromethyl, ethyl, n-propyl, 2-fluoroethyl, 2-chloroethyl, ethynyl or

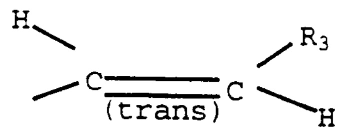

wherein $R_3$ is chloro, bromo, iodo, hydrogen, methyl or trifluoromethyl; $R_4$ is alkyl; $R_5$ is hydrogen, alkyl, substituted alkyl, or aryl; and $R_6$ and $R_7$ are independently hydrogen $-PO_3H_2$ or

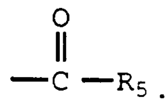

**2.** A compound according to Claim 1 wherein $R_1$ is

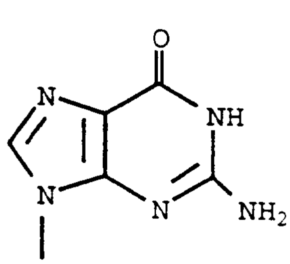 , 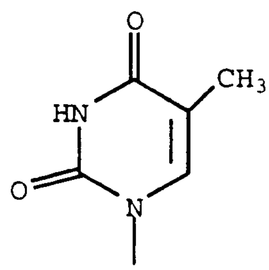 ,

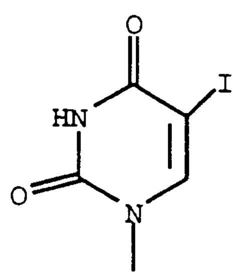 , 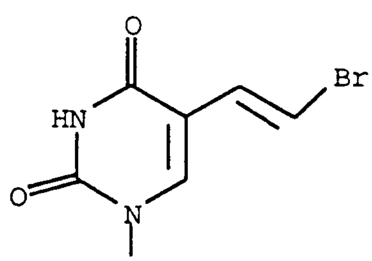 ,

or 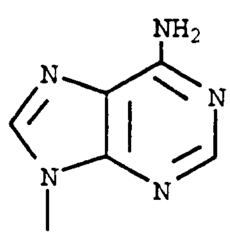 .

**3.** A compound according to Claim 2 wherein $R_6$ and $R_7$ are hydrogen.

**4.** A compound according to Claim 3 wherein $R_1$ is

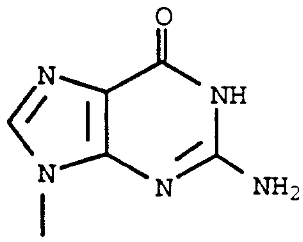 , 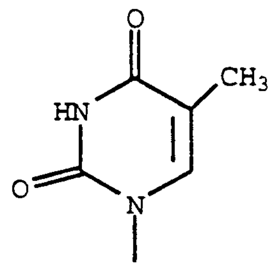 ,

or 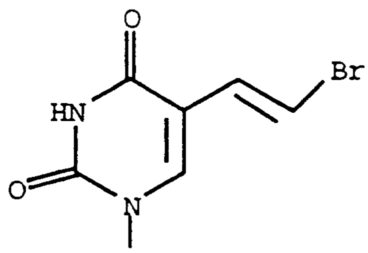 .

**5.** A compound according to Claim 1, (1α,3β,4α)-2-amino-1,9-dihydro-9-[3-hydroxy-4-(hydroxymethyl)-2-methylenecyclopentyl]-6H-purin-6-one.

**6.** A compound according to Claim 1, (1α,3β,4α)-4-(6-amino-9H-purin-9-yl)-2-hydroxy-3-methylenecyclopentanemethanol.

**7.** A compound according to Claim 1, (1α,3β,4α)-1-[3-hydroxy-4-(hydroxymethyl)-2-methylenecyclopentyl]-5-methyl-2,4(1H,3H)-pyrimidinedione.

**8.** A compound according to Claim 1, (1α,3β,4α)-1-[3-hydroxy-4-(hydroxymethyl)-2-methylenecyclopentyl]-5-iodo-2,4(1H,3H)-pyrimidinedione.

**9.** A compound according to Claim 1, [1α(E),3β,4α]-5-[2-bromoethenyl)-1-[3-hydroxy-4-(hydroxymethyl)-2-methylenecyclopentyl]-2,4(1H,3H)-pyrimidinedione.